# EUROPEAN PATENT SPECIFICATION

(11) **EP 2 190 875 B1**
(45) Date of publication and mention of the grant of the patent: **10.07.2019**
(21) Application number: 08767415.6
(22) Date of filing: 23.04.2008
(51) Int. Cl.: C12N 9/00, C07H 21/04, C12N 15/00, A61K 39/395, C07K 16/10, C07K 16/12, C07K 16/18

(54) **Catalytic Antibodies against HIV gp120**
Katalytische Antikörper gegen HIV gp120
Anticorps catalytiques contre le VIH gp120

(30) Priority: 23.04.2007 US 913335 P
(43) Date of publication of application: 02.06.2010
(73) Proprietor: Paul, Sudhir, Missouri City, TX 77459 (US); Planque, Stephanie, Houston, TX 77054 (US); Brown, Eric L., Houston, TX 77030 (US); Smith, Keri C., Houston, TX 77009 (US); Nishiyama, Yasuhiro, Houston, TX 77030 (US); Taguchi, Hiroaki, Houston, TX 77030 (US)
(72) Inventor: Paul, Sudhir, Missouri City, TX 77459 (US); Planque, Stephanie, Houston, TX 77054 (US); Brown, Eric L., Houston, TX 77030 (US); Smith, Keri C., Houston, TX 77009 (US); Nishiyama, Yasuhiro, Houston, TX 77030 (US); Taguchi, Hiroaki, Houston, TX 77030 (US)
(74) Representative: Schneiter, Sorin
(86) International application number: PCT/US2008/005221
(87) International publication number: WO 2009/023043

(56) References cited:
- WO-A1-97/03696
- WO-A2-2004/003019
- WO-A2-2004/087738
- KARLE SANGEETA ET AL: "Cross-clade HIV-1 neutralization by an antibody fragment from a lupus phage display library.", AIDS (HAGERSTOWN), vol. 18, no. 2, 23 January 2004 (2004-01-23), pages 329-331, XP002695864, ISSN: 0269-9370
- ZHOU Y-X ET AL: "Prospects for immunotherapeutic proteolytic antibodies", JOURNAL OF IMMUNOLOGICAL METHODS, ELSEVIER SCIENCE PUBLISHERS B.V.,AMSTERDAM, NL, vol. 269, no. 1-2, 1 November 2002 (2002-11-01), pages 257-268, XP004387971, ISSN: 0022-1759, DOI: 10.1016/S0022-1759(02)00236-3
- NISHIYAMA Y ET AL: "Antibodies to the superantigenic site of HIV-1 gp120: Hydrolytic and binding activities of the light chain subunit", MOLECULAR IMMUNOLOGY, PERGAMON, GB, vol. 44, no. 10, 1 April 2007 (2007-04-01) , pages 2707-2718, XP026863227, ISSN: 0161-5890 [retrieved on 2007-02-14]
- TAGUCHI H ET AL: "A MECHANISM-BASED PROBE FOR GP120-HYDROLYZING ANTIBODIES", BIOORGANIC & MEDICINAL CHEMISTRY LETTERS, PERGAMON, ELSEVIER SCIENCE, GB, vol. 12, no. 21, 1 November 2002 (2002-11-01), pages 3167-3170, XP008060876, ISSN: 0960-894X, DOI: 10.1016/S0960-894X(02)00640-6
- NESHAT M N ET AL: "Mapping the B cell superantigen binding site for HIV-1 gp120 on a V(H)3 Ig", INTERNATIONAL IMMUNOLOGY, OXFORD UNIVERSITY PRESS, GB, vol. 12, no. 3, 1 March 2000 (2000-03-01), pages 305-312, XP002455647, ISSN: 0953-8178, DOI: 10.1093/INTIMM/12.3.305
- PAUL S ET AL: "SPECIFIC HIV GP120-CLEAVING ANTIBODIES INDUCED BY COVALENTLY REACTIVE ANALOG OF GP120", JOURNAL OF BIOLOGICAL CHEMISTRY, AMERICAN SOCIETY FOR BIOCHEMISTRY AND MOLECULAR BIOLOGY, US, vol. 278, no. 22, 28 March 2003 (2003-03-28), pages 20429-20435, XP008060874, ISSN: 0021-9258, DOI: 10.1074/JBC.M300870200
- PAUL S. ET AL.: 'Natural catalytic antibodies: peptide-hydrolyzing activities of Bence Jones proteins and VL fragment' JOURNAL OF BIOLOGICAL CHEMISTRY vol. 270, no. 25, 23 June 1995, pages 15257 - 15261, XP002385361
- NIESNER U. ET AL.: 'Quantitation of the tumor-targeting properties of antibody fragments conjugated to cell-permeating HIV-1 TAT peptides' BIOCONJUGATION CHEMISTRY vol. 13, no. 4, July 2002 - August 2002, pages 729 - 736, XP008131558
- MUYLDERMANS S.: 'Single domain camel antibodies: current status' JOURNAL OF BIOTECHNOLOGY vol. 74, no. 4, June 2001, pages 277 - 302, XP008019929
- NOVOTNY J. AND HABER E.: 'Structural invariants of antigen binding: Comparison of immunoglobulin VL-VH and VL-VL domain dimers' PROCEEDINGS OF NATIONAL ACADEMY OF SCIENCES, USA vol. 82, July 1985, pages 4592 - 4596, XP008131559
- Srinath Kasturi Rangan ET AL: "Degradation of [beta]-Amyloid by Proteolytic Antibody Light Chains +", Biochemistry, vol. 42, no. 48, 1 December 2003 (2003-12-01), pages 14328-14334, XP055353654, US ISSN: 0006-2960, DOI: 10.1021/bi035038d
- Srinath Kasturi Rangan ET AL: "Degradation of [beta]-Amyloid by Proteolytic Antibody Light Chains +", Biochemistry, vol. 42, no. 48, 1 December 2003 (2003-12-01), pages 14328-14334, XP055353656, US ISSN: 0006-2960, DOI: 10.1021/bi035038d
- LACROIX-DESMAZES SEBASTIEN ET AL: "High levels of catalytic antibodies correlate with favorable outcome in sepsis", PROCEEDINGS NATIONAL ACADEMY OF SCIENCES PNAS, NATIONAL ACADEMY OF SCIENCES, US, vol. 102, no. 11, 15 March 2005 (2005-03-15), pages 4109-4113, XP002582358, ISSN: 0027-8424, DOI: 10.1073/PNAS.0500586102 [retrieved on 2005-03-02]
- Anonymous: "IC50 - Wikipedia, the free encyclopedia", , 10 May 2016 (2016-05-10), XP055288105, Retrieved from the Internet: URL:https://en.wikipedia.org/wiki/IC50 [retrieved on 2016-07-13]
- B. Joos ET AL: "Long-Term Multiple-Dose Pharmacokinetics of Human Monoclonal Antibodies (MAbs) against Human Immunodeficiency Virus Type 1 Envelope gp120 (MAb 2G12) and gp41 (MAbs 4E10 and 2F5)", Antimicrobial agents and chemotherapy, vol. 50, no. 5, 1 May 2006 (2006-05-01), pages 1773-1779, XP055170370, ISSN: 0066-4804, DOI: 10.1128/AAC.50.5.1773-1779.2006
- PAUL S ET AL: "Natural Catalytic Antibodies: Peptide-hydrolyzing Activities of Bence Jones Proteins and V-L Fragment", JOURNAL OF BIOLOGICAL CHEMISTRY, AMERICAN SOCIETY FOR BIOCHEMISTRY AND MOLECULAR BIOLOGY, US, vol. 270, no. 25, 23 June 1995 (1995-06-23), pages 15257-15261, XP002385361, ISSN: 0021-9258, DOI: 10.1074/JBC.270.25.15257
- Stephanie Planque ET AL: "Characterization of gp120 Hydrolysis by IgA Antibodies from Humans without HIV Infection", AIDS Research and Human Retroviruses, vol. 23, no. 12, 1 December 2007 (2007-12-01), pages 1541-1554, XP055039485, ISSN: 0889-2229, DOI: 10.1089/aid.2007.0081
- SUDHIR PAUL ET AL: "Naturally Occurring Proteolytic Antibodies: SELECTIVE IMMUNOGLOBULIN M-CATALYZED HYDROLYSIS OF HIV gp120", JOURNAL OF BIOLOGICAL CHEMISTRY, AMERICAN SOCIETY FOR BIOCHEMISTRY AND MOLECULAR BIOLOGY, US, vol. 279, no. 38, 21 July 2004 (2004-07-21), pages 39611-39619, XP008153850, ISSN: 0021-9258, DOI: 10.1074/JBC.M406719200

## Description

### FIELD OF THE INVENTION

This invention relates to the fields of biochemistry, immunology, molecular biology and medicine. More specifically, the invention relates to preparation and use of immunoglobulins and fragments thereof with the ability to hydrolyze or bind to HIV gp120.

### BACKGROUND OF THE INVENTION

Some immunoglobulins (Igs) have the ability to catalyze chemical reactions through the binding of an antigen, its chemical transformation and the conversion and release of one or more products. Transform of the chemical structure of antigens by such Igs can induce permanent inactivation of antigens. A single catalytic Ig molecule can hydrolyze thousands of antigen molecules over its biological lifetime, which enhances the biological potency of the catalyst compared to a stoichiometrically binding antibody. Catalytic Igs, therefore, can be developed as potent therapeutic agents capable of removing harmful polypeptide and other classes of antigens. Some Igs contain catalytic sites in their variable (V) domains that have properties similar to the catalytic sites of conventional serine protease class of enzymes. Igs with esterolytic and proteolytic activity have been reported [1-5]. Similarly, some Igs hydrolyze nucleic acids [6,7].

An appreciation of the structural organization of Igs is helpful in understanding the scope of the present invention. A brief review of this aspect follows. Generally, Igs contain light (L) chain and heavy (H) chain subunits. The V domains of these subunits contain the antigen binding site (paratope). Contacts with conventional antigenic epitopes occur mainly at the complementarity determining regions (CDRs) and to a lesser extent the framework regions (FR) of the V domains. The human Ig repertoire, defined as the number of Igs with different antigen binding site structures is estimated at 10¹¹-10¹². V domain diversity is generated by the following processes: (a) inheritance of about 50 germline genes each encoding the V domains of L and H subunits; (b) combinatorial diversity brought about by assembly of different L and H chains within the quartenary structure of Igs; (c) junctional diversity generated during recombination of the V and joining (J) gene segments of the L chain, and the V, diversity (D) and J gene segments of the H chain; and (d) rapid mutation occurring in the CDRs over the course of B cell clonal selection, a process entailing antigen binding to Igs expressed as components of the B cell receptor (BCR), and resulting in stimulation of division of the B cells expressing BCRs with the highest binding affinity. An additional level of diversity is offered by the use of different constant domains by Igs, that is, the µ, δ, γ, and α regions of the H chain and the κ and λ chains of the L chain. Early in the ontogeny of the immune response, Igs contain µ or δ constant regions. Later, isotype switching occurs, and the µ/δ regions are replaced by γ/α/ε in more differentiated Igs.

Various advances of technology in monoclonal and recombinant Ig techniques have accelerated the identification, selection and purification of catalytic Ig species. One approach to generating catalytic Igs involves immunizing an animal with a stable analog of the transition state of the reaction to be catalyzed and screening for Igs that bind more strongly to the transition state analog than to the corresponding substrate. The Igs, like enzymes, have a site that is complementary to the 3-D and ionic structure of the transition state analog. A large number of Igs synthesized in response to immunization with a transition state analog can bind the analog, but only a small minority will catalyze the reaction of interest. For example, only one catalytic Ig may be found for every 100-1,000 Igs screened. Another major challenge is that the catalytic activity of the Igs can be low compared to the naturally occurring enzymes, usually by a factor of 10³ or more.

To be medically useful, the catalytic Ig must also be specific for the desired target antigen. While promiscuous catalytic Igs capable of hydrolyzing polypeptides are common [5,8-10], specific catalytic Igs directed to medically important target proteins are rare.

Advances in the field of catalytic Igs, therefore, are dependent on identifying Igs that have high level catalytic activity and the correct epitope specificity enabling specific catalysis directed against the target antigen. The foregoing problems have been recognized for many years. Numerous solutions have been proposed, but none adequately address the problems that must be solved in isolating Igs that have the ability to specifically catalyze medically important biochemical reactions. Renewable and homogeneous sources of well-characterized catalytic Igs are needed for medical applications. A brief review of Ig technologies that may be useful in isolating catalytic Igs follows.

Traditional methods to clone Igs from humans consist of immortalizing lymphocytes derived from peripheral blood (or lymphoid tissues obtained by surgery), for example by transformation with Epstein Barr virus followed by fusion with a myeloma cell lines. The resultant hybridoma cell lines are screened for production of the desired Abs, for example by measuring the binding to a specific antigen by ELISA.

Methods are also available to clone the expressed Ig V domain repertoires in the form of libraries displayed on a suitable surface. The Ab fragments can be cloned as Fab fragments, single chain Fv (scFv) fragments or the L chain subunits. Fab and scFv constructs usually reproduce faithfully the binding activity of full-length Abs (e.g., [11]). Previous reports have documented the antigen binding activity of L chain subunit independent of its H chain partner, albeit at reduced strengths compared to native Abs [12,13]. The V domains of the scFv fragments are usually linked by flexible peptide linkers. Cloning of V domain repertoires is usually accomplished by recovering mRNA from lymphocytes and amplification by the reverse transcriptase-polymerase chain reaction. Mixtures of primers are employed to capture as large a proportion of the expressed repertoire as possible. The primers anneal to comparatively conserved FR1 and FR4 nucleotide stretches located at the 5' and 3' ends of the V domains, respectively, allowing amplification of V domains belonging diverse V gene families. To obtain expressible scFv constructs, the VL and VH domains are cloned into a suitable vector containing a short flexible peptide and an inducible promoter. Peptide tags such as the his6 tag are incorporated into the protein to enable rapid purification by metal affinity chromatography. The length and constitution of the peptide linker is an important variable in ensuring the appropriate intramolecular VL-VH interactions.

The next task is to isolate the minority of individual antigen combining sites with the desired antigen recognition characteristics. This can be accomplished using display technologies [14]. Vectors permitting display of recombinant proteins on the surfaces of phages, retroviruses, bacteria and yeast have been developed. For example, fusion proteins composed of Ig fragments linked to a phage coat protein are expressed from phagemid or phage vectors in bacteria. The recombinant phages display Ig fragments on their surface. The packaged phages contain single stranded DNA encoding the Ig fusion protein. Fractionation of phages based on binding to immobilized antigen yields, therefore, the VL/VH genes of Abs with the desired specificity. Phagemid vectors are useful because a codon at the junction of the Ab and phage coat protein genes is read as a sense codon by bacteria employed to package phages and as a stop codon by bacteria employed to obtain soluble Ab fragments free of the phage coat protein sequence.

Immunotherapeutic agents should preferably have a long half-life to avoid repeated infusions. The half-life of full-length IgG in human circulation is 2-3 weeks, compared to half-lives on the order of minutes for scFv constructs and free Ig L chain subunits. Therefore, various strategies have been developed to increase the stability of Ig fragments in vivo. Examples are the inclusion of a polyethylene glycol molecule at the Ig fragment terminus [15,16] or linkage to the constant region of Igs (Fc) [17]. The V domains can also be routinely recloned in vectors containing the constant domains of heavy and light chains. Expression of these vectors in suitable mammalian cells yields full-length Igs with increased half-life in vivo [18].

The properties of the target antigen are important in the success of medical applications of catalytic Igs. The targeted antigen can be chosen for Ig targeting based on the principles. First, the antigen should fulfill a pathogenic role. For example, the targeted antigen may interfere in some essential endogenous cellular or metabolic function. Alternatively, in the case of microbial antigens, the antigen should be important for growth of the microbe or it may be important in diverting host immune responses away from protection against the microbe. Second, removal of the antigen by Igs should not be associated with a deleterious side effect. This is particularly important in targeting of an endogenous antigen by Igs, e.g., amyloid β peptide in Alzheimer disease, as most endogenous antigens fulfill useful biological functions. In the case of microbial antigens, the danger of cross-reaction with endogenous antigens should be minimized. Immune complexes of antigens with Igs containing Fc regions have the potential of reacting with Fc receptors expressed on inflammatory cells and inducing undesirable inflammatory reactions. This danger is minimized if the Ig has catalytic activity, as the longevity of the immune complexes is reduced due to antigen chemical transformation and product release.

### Examples of antigens suitable for Ig targeting are presented below.

*Amyloid β peptide (Aβ)*. Aβ is the target of conventional non-catalytic Igs in ongoing clinical trials for the treatment of Alzheimer disease. A monoclonal IgG [19] and pooled polyclonal IgG from healthy humans [20] are under trial. The rationale for Aβ targeting is as follows. In 1907 that the first pathological lesion associated with dementia, the cerebral plaque, was reported by Alzheimer [21]. The cerebral lesion was called an "amyloid" plaque because iodine, which stained the cerebral plaque, also stains starch. The true chemical composition of the "amyloid" plaque was elusive until 1984 when Glenner and Wang discovered a means to solubilize the cerebral plaques in Alzheimer's disease (AD) and showed that they are composed principally of peptides Aβ1-40 and Aβ1-42. These peptides are identical but for the two additional amino acids, Ile and Ala, at the C-terminus of Aβ1-42 [22]. Both peptides are derived by proteolytic processing of the larger amyloid precursor protein (APP), which is composed of 770 amino acids and has the characteristics of a transmembrane protein [23]. APP is cleaved by β and γ secretases, releasing the Aβ peptides [24,25]. The role of Aβ peptides in the pathogenesis of AD is supported by findings that: (a) Familial AD is associated with mutations in the APP gene or the secretase genes; (b) Transgenic mice, expressing mutant human, APP genes develop an age-associated increase in cerebral Aβ peptides and amyloid plaques as well as cognitive decline [26,27]; (c) Mutant, human APP-tg mice that do not process APP to Aβ show no cognitive decline, suggesting that increased Aβ peptides and not the mutant APP is responsible for cognitive decline [28,29]; and (d) Synthetic Aβ peptides and their oligomers are neurotoxic *in vitro* [30,31].

*HIV gp120.* Igs directed to the HIV coat glycoprotein gp120 have long been under consideration for immunotherapy of HIV infection. A key step in HIV infection is the binding of gp120 to host cell CD4 receptors. Additionally, gp120 plays a significant role in viral propagation and demonstrates a toxic effect on cells that are not infected with HIV [32,33]. gp120 is toxic for neurons, it facilitates lyses of lympocytes by an antibody-dependent mechanism, and it increases the binding of complement components to cells [34-39]. It has been shown that monoclonal Igs can bind the CD4 binding site (e.g., [40,41]). However, gp120 expresses many antigenic epitopes, and the immunodominant epitopes are located in the variable regions of gp120. Igs to the immunodominant epitopes do not neutralize diverse strains with varying sequence of the variable gp120 regions. Igs to the conserved gp120 sequences are necessary for therapy of HIV infection. Such Igs can also be used as topical microbicides to prevent vaginal and rectal transmission of HIV via sexual intercourse. gp120 contains a B cell superantigenic epitopes, defined as an antigenic epitope to which Igs are present in the preimmune repertoire without the requirement of adaptive immune specialization [42]. Residues 421-433 of this epitope are also important in CD4 host receptor binding [43,44]. Igs to the superantigenic epitope hold the potential of neutralizing HIV broadly. However, superantigens are thought to be recognized mainly by conserved regions of Ig V domains, including the conserved regions of the FRs and CDRs. Adaptive improvement of the superantigen recognition function appears to be difficult. No monoclonal or polyclonal Igs with the ability to neutralize the entire range of HIV strains belonging to various clades are available. WO2004087738 discloses gp120 binding and HIV neutralizing activity of single Fv constructs screened from a library obtained from lupus patients.

*Staphylococcus aureus.* This bacterium is an opportunistic pathogen that colonizes the skin (primarily the anterior nasal vestibule) of approximately 30-50% (with 20-30% persistently colonized) of the population without causing clinical disease symptoms [45,46]. Persistently colonized individuals are designated 'carriers'. Such individuals are designated 'carriers'. A minority of humans harboring the bacterium develop clinical disease ranging from minor skin infections to lethal infections associated with abscess formation, endocarditis and pneumonia. Certain antibiotics are available for the treatment of *S. aureus-caused* disease, but the number of antibiotic-resistant strains is increasing rapidly. Host immunological factors play a role in determining susceptibility to initial colonization and development of *S. aureus* disease. However, certain virulence factors produced by *S. aureus* can downregulate host immune defenses profoundly, helping the bacterium colonize various anatomic sites and cause serious disease. The protective effect of Igs directed against *S. aureus* antigens has been reported in several experimental studies [47-49]. Persistent antigenic exposure in *S. aureus* carriers can be hypothesized to induce adaptive synthesis of protective Igs. An insufficient adaptive response may be a predisposing factor in progression of infection. As noted above, certain microbial antigens behave as B cell superantigens. If *S. aureus* proteins have superantigenic character, protective Igs to the bacterium may be produced spontaneously without prior infection. *S. aureus* produces a host of virulence factors important in bacterial adhesion, toxicity for host cells and modulation of the host immune syste. Selected *S. aureus* suitable for targeting by Igs are listed in Table 1.

**Table 1. Example S. aureus proteins suitable for targeting by Igs**

| *S. aureus* proteins | Function |
|---|---|
| Efb | Immune Impairment |
| Protein A | Immune Evasion |
| Map19 (Eap) | Immune Impairment |
| ClfA₂₂₉₋₅₄ | Fibrinogen/Fibronectin Adhesin |
| ClfB₂₀₁₋₅₄₂ | Fibrinogen/Cytokeratin Adhesin |
| FnbpA | Fibronectin Adhesin |
| CAN | Collagen Adhesin |
| SdrE₅₁₋₆₀₆ | Potential Adhsin |
| Alpha toxin | Toxin |
| LukF | Toxin |
| LukS | Toxin |

*Hepatitis C virus (HCV).* HCV infection leads to chronic hepatitis, liver failure and hepatocellular carcinoma. It is estimated that over 170 million people worldwide are infected with HCV with the majority leading to chronic disease [50]. HCV genotypes 1a, 1b, 2a and 2b are common in the United States. Liver transplantation often is necessary due to liver cirrhosis secondary to viral infection. The mainstay of current therapy is a combination of interferon and ribavirin, which leads to clinical improvement in a subpopulation of patients with chronic HCV infection. Clearly, more strategies are needed for treatment and prevention of HCV infection [51]. The E2 coat protein expressed by HCV is thought to be essential for viral infection by virtue of its role in host cell binding [52]. E2 contains hypervariable regions and comparatively conserved regions. Igs to the hypervariable regions are frequent in infected individuals [53]. Conventional non-catalytic Igs to E2 have been suggested to be important in control of virus infection [53]. Certain monoclonal Igs to E2 neutralize the virus and [54] are under consideration for therapy of HCV infection.

### References

1. Sun M, Gao QS, Li L, Paul S: Proteolytic activity of an antibody light chain. J Immunol 1994, 153:5121-5126.
2. Paul S: Catalytic activity of anti-ground state antibodies, antibody subunits, and human autoantibodies. Appl Biochem Biotechnol 1994, 47:241-253; discussion 253-245.
3. Li L, Sun M, Gao QS, Paul S: Low level formation of potent catalytic IgG fragments mediated by disulfide bond instability. Mol Immunol 1996, 33:593-600.
4. Li L, Paul S, Tyutyulkova S, Kazatchkine MD, Kaveri S: Catalytic activity of anti-thyroglobulin antibodies. J Immunol 1995, 154:3328-3332.
5. Kalaga R, Li L, O'Dell JR, Paul S: Unexpected presence of polyreactive catalytic antibodies in IgG from unimmunized donors and decreased levels in rheumatoid arthritis. J Immunol 1995, 155:2695-2702.
6. Shuster AM, Gololobov GV, Kvashuk OA, Bogomolova AE, Smirnov IV, Gabibov AG: DNA hydrolyzing autoantibodies. Science 1992, 256:665-667.
7. Matsuura K, Ikoma S, Yoshida K, Sinohara H: DNA-hydrolyzing activity of Bence Jones proteins. Biochem Biophys Res Commun 1998, 243:719-721.
8. Paul S, Li L, Kalaga R, Wilkins-Stevens P, Stevens FJ, Solomon A: Natural catalytic antibodies: peptide-hydrolyzing activities of Bence Jones proteins and VL fragment. J Biol Chem 1995, 270:15257-15261.
9. Planque S, Bangale Y, Song XT, Karle S, Taguchi H, Poindexter B, Bick R, Edmundson A, Nishiyama Y, Paul S: Ontogeny of proteolytic immunity: IgM serine proteases. J Biol Chem 2004, 279:14024-14032.
10. Mitsuda Y, Planque S, Hara M, Kyle R, Taguchi H, Nishiyama Y, Paul S: Naturally occurring catalytic antibodies: evidence for preferred development of the catalytic function in IgA class antibodies. Mol Biotechnol 2007, 36:113-122.
11. Pantoliano MW, Bird RE, Johnson S, Asel ED, Dodd SW, Wood JF, Hardman KD: Conformational stability, folding, and ligand-binding affinity of single-chain Fv immunoglobulin fragments expressed in Escherichia coli. Biochemistry 1991, 30:10117-10125.
12. Masat L, Wabl M, Johnson JP: A simpler sort of antibody. Proc Natl Acad Sci U S A 1994, 91:893-896.
13. Sun M, Li L, Gao QS, Paul S: Antigen recognition by an antibody light chain. J Biol Chem 1994, 269:734-738.
14. Marks JD, Hoogenboom HR, Bonnert TP, McCafferty J, Griffiths AD, Winter G: Bypassing immunization. Human antibodies from V-gene libraries displayed on phage. J Mol Biol 1991, 222:581-597.
15. Kubetzko S, Balic E, Waibel R, Zangemeister-Wittke U, Pluckthun A: PEGylation and multimerization of the anti-p185HER-2 single chain Fv fragment 4D5: effects on tumor targeting. J Biol Chem 2006, 281:35186-35201.
16. Krinner EM, Hepp J, Hoffmann P, Bruckmaier S, Petersen L, Petsch S, Parr L, Schuster I, Mangold S, Lorenczewski G, et al.: A highly stable polyethylene glycol-conjugated human single-chain antibody neutralizing granulocyte-macrophage colony stimulating factor at low nanomolar concentration. Protein Eng Des Sel 2006, 19:461-470.
17. Yang K, Basu A, Wang M, Chintala R, Hsieh MC, Liu S, Hua J, Zhang Z, Zhou J, Li M, et al.: Tailoring structure-function and pharmacokinetic properties of single-chain Fv proteins by site-specific PEGylation. Protein Eng 2003, 16:761-770.
18. McLean GR, Nakouzi A, Casadevall A, Green NS: Human and murine immunoglobulin expression vector cassettes. Mol Immunol 2000, 37:837-845.
19. DeMattos RB, Bales KR, Cummins DJ, Dodart JC, Paul SM, Holtzman DM: Peripheral anti-A beta antibody alters CNS and plasma A beta clearance and decreases brain A beta burden in a mouse model of Alzheimer's disease. Proc Natl Acad Sci U S A 2001, 98:8850-8855.
20. Dodel RC, Du Y, Depboylu C, Hampel H, Frolich L, Haag A, Hemmeter U, Paulsen S, Teipel SJ, Brettschneider S, et al.: Intravenous immunoglobulins containing antibodies against beta-amyloid for the treatment of Alzheimer's disease. J Neurol Neurosurg Psychiatry 2004, 75:1472-1474.
21. Alzheimer A: Uber eine eigenartige Erkrankung der Hirnrinde. Allg. Z. Psychiat. 1907, 64:146-148.
22. Glenner GG, Wong CW: Alzheimer's disease and Down's syndrome: sharing of a unique cerebrovascular amyloid fibril protein. Biochem Biophys Res Commun 1984,122:1131-1135.
23. Kang J, Lemaire HG, Unterbeck A, Salbaum JM, Masters CL, Grzeschik KH, Multhaup G, Beyreuther K, Muller-Hill B: The precursor of Alzheimer's disease amyloid A4 protein resembles a cell-surface receptor. Nature 1987, 325:733-736.
24. Haass C, Schlossmacher MG, Hung AY, Vigo-Pelfrey C, Mellon A, Ostaszewski BL, Lieberburg I, Koo EH, Schenk D, Teplow DB, et al.: Amyloid beta-peptide is produced by cultured cells during normal metabolism. Nature 1992, 359:322-325.
25. Seubert P, Vigo-Pelfrey C, Esch F, Lee M, Dovey H, Davis D, Sinha S, Schlossmacher M, Whaley J, Swindlehurst C, et al.: Isolation and quantification of soluble Alzheimer's beta-peptide from biological fluids. Nature 1992, 359:325-327.
26. Games D, Adams D, Alessandrini R, Barbour R, Berthelette P, Blackwell C, Carr T, Clemens J, Donaldson T, Gillespie F, et al.: Alzheimer-type neuropathology in transgenic mice overexpressing V717F beta-amyloid precursor protein. Nature 1995, 373:523-527.
27. Morgan D, Diamond DM, Gottschall PE, Ugen KE, Dickey C, Hardy J, Duff K, Jantzen P, DiCarlo G, Wilcock D, et al.: A beta peptide vaccination prevents memory loss in an animal model of Alzheimer's disease. Nature 2000, 408:982-985.
28. Citron M, Oltersdorf T, Haass C, McConlogue L, Hung AY, Seubert P, Vigo-Pelfrey C, Lieberburg I, Selkoe DJ: Mutation of the beta-amyloid precursor protein in familial Alzheimer's disease increases beta-protein production. Nature 1992, 360:672-674.
29. Ohno M, Sametsky EA, Younkin LH, Oakley H, Younkin SG, Citron M, Vassar R, Disterhoft JF: BACE1 deficiency rescues memory deficits and cholinergic dysfunction in a mouse model of Alzheimer's disease. Neuron 2004, 41:27-33.
30. Solomon B, Koppel R, Frankel D, Hanan-Aharon E: Disaggregation of Alzheimer beta-amyloid by site-directed mAb. Proc Natl Acad Sci U S A 1997, 94:4109-4112.
31. Chromy BA, Nowak RJ, Lambert MP, Viola KL, Chang L, Velasco PT, Jones BW, Fernandez SJ, Lacor PN, Horowitz P, et al.: Self-assembly of Abeta(1-42) into globular neurotoxins. Biochemistry 2003, 42:12749-12760.
32. Kido H, Kamoshita K, Fukutomi A, Katunuma N: Processing protease for gp160 human immunodeficiency virus type I envelope glycoprotein precursor in human T4+ lymphocytes. Purification and characterization. J Biol Chem 1993, 268:13406-13413.
33. Capon DJ, Ward RH: The CD4-gp120 interaction and AIDS pathogenesis. Annu Rev Immunol 1991, 9:649-678.
34. Gelderblom HR, Reupke H, Pauli G: Loss of envelope antigens of HTLV-III/LAV, a factor in AIDS pathogenesis? Lancet 1985, 2:1016-1017.
35. Brenneman DE, Westbrook GL, Fitzgerald SP, Ennist DL, Elkins KL, Ruff MR, Pert CB: Neuronal cell killing by the envelope protein of HIV and its prevention by vasoactive intestinal peptide. Nature 1988, 335:639-642.
36. Muller WE, Schroder HC, Ushijima H, Dapper J, Bormann J: gp120 of HIV-1 induces apoptosis in rat cortical cell cultures: prevention by memantine. Eur J Pharmacol 1992, 226:209-214.
37. Hober D, Jewett A, Bonavida B: Lysis of uninfected HIV-1 gp120-coated peripheral blood-derived T lymphocytes by monocyte-mediated antibody-dependent cellular cytotoxicity. FEMS Immunol Med Microbiol 1995, 10:83-91.
38. Laurent-Crawford AG, Coccia E, Krust B, Hovanessian AG: Membrane-expressed HIV envelope glycoprotein heterodimer is a powerful inducer of cell death in uninfected CD4+ target cells. Res Virol 1995, 146:5-17.
39. Stoiber H, Ebenbichler C, Schneider R, Janatova J, Dierich MP: Interaction of several complement proteins with gp120 and gp41, the two envelope glycoproteins of HIV-1. Aids 1995, 9:19-26.
40. Thali M, Olshevsky U, Furman C, Gabuzda D, Posner M, Sodroski J: Characterization of a discontinuous human immunodeficiency virus type 1 gp120 epitope recognized by a broadly reactive neutralizing human monoclonal antibody. J Virol 1991, 65:6188-6193.
41. Thali M, Furman C, Ho DD, Robinson J, Tilley S, Pinter A, Sodroski J: Discontinuous, conserved neutralization epitopes overlapping the CD4-binding region of human immunodeficiency virus type 1 gp120 envelope glycoprotein. J Virol 1992, 66:5635-5641.
42. Berberian L, Goodglick L, Kipps TJ, Braun J: Immunoglobulin VH3 gene products: natural ligands for HIV gp120. Science 1993, 261:1588-1591.
43. Karray S, Zouali M: Identification of the B cell superantigen-binding site of HIV-1 gp120. Proc Natl Acad Sci U S A 1997, 94:1356-1360.
44. Goodglick L, Zevit N, Neshat MS, Braun J: Mapping the Ig superantigen-binding site of HIV-1 gp120. J Immunol 1995, 155:5151-5159.
45. Kissane JM: Staphylococcal Infections. In Pathology of Infectious Diseases. Edited by Connor DH, Chandler FW, Schwartz HJ, Manz HJ, Lack EE: Appleton and Lange; 1997:805-816. vol I.]
46. Lowy FD: Staphylococcus aureus infections. N. Engl. J. Med. 1998, 339:520-532.
47. Mamo W, Boden M, Flock JI: Vaccination with Staphylococcus aureus fibrinogen binding proteins (FgBPs) reduces colonisation of S. aureus in a mouse mastitis model. FEMS Immunol Med Microbiol 1994, 10:47-53.
48. Lee JC: Development of Antistaphylococcal Vaccines. Curr Infect Dis Rep 2001, 3:517-524.
49. Schaffer AC, Solinga RM, Cocchiaro J, Portoles M, Kiser KB, Risley A, Randall SM, Valtulina V, Speziale P, Walsh E, et al.: Immunization with Staphylococcus aureus clumping factor B, a major determinant in nasal carriage, reduces nasal colonization in a murine model. Infect Immun 2006, 74:2145-2153.
50. Centers for Disease Control and Prevention. Disease burden from viral hepatitis A, B and C in the United States. URL: http://www.cdc.gov/nicdod/diseases/hepatitis/heptab3. htm 1997.
51. Dusheiko G: Side effects of interferon alpha in viral hepatitis. NIH Consensus Development Conference on Management of Hepatitis C 1997, Program and Abstracts IV:105-112.
52. Barth H, Liang TJ, Baumert TF: Hepatitis C virus entry: molecular biology and clinical implications. Hepatology 2006, 44:527-535.
53. Zeisel MB, Fafi-Kremer S, Fofana I, Barth H, Stoll-Keller F, Doffoel M, Baumert TF: Neutralizing antibodies in hepatitis C virus infection. World J Gastroenterol 2007, 13:4824-4830.
54. Galun E, Terrault NA, Eren R, Zauberman A, Nussbaum O, Terkieltaub D, Zohar M, Buchnik R, Ackerman Z, Safadi R, et al.: Clinical evaluation (Phase I) of a human monoclonal antibody against hepatitis C virus: safety and antiviral activity. J Hepatol 2007, 46:37-44.

### SUMMARY OF THE INVENTION

The present invention provides an isolated or purified variable (V) domain construct comprising two individual immunoglobulin (Ig) V domains, wherein said construct comprises the light chain V (VL) domain GL2-VL having SEQ ID NO: 47 and an Ig heavy chain V (VH) domain comprising a sequence selected from the group consisting of GL2-FR1m having SEQ ID NO: 64, GL2-FR3m having SEQ ID NO: 65, and GL2-CDR2m having SEQ ID NO: 68.

Optionally, the V domain construct can contain peptide or non-peptide structures that direct the IgV to the desired target anatomic site. For example, inclusion of polyanionic compound such as a putrescine in a peptide tag can facilitate passage of the IgV across the blood-brain-barrier.

In an embodiment of the invention concerns recognition of B cell superantigens by the V domains, which is thought to occur mainly at conserved V domain residues. Swapping of the framework regions (FRs) or complementarity determining regions (CDRs) of the VH domains by corresponding FRs or CDRs drawn from other V domains is shown to improve B cell superantigen recognition capability. Similarly, mutations introduced at individual amino acids in the FRs or CDRs can be employed to improve the ability of IgVs to recognize B cell superantigens.

The IgVs can be engineered further to improve their stability in vascular circulation and other anatomic sites, for example by linkage to a polyethylene glycol molecule or the Fc region of Igs.

In an aspect, the invention provides a monoclonal full-length immunoglobulin-like molecule with γ, α or µ class constant domains comprising the two V domains of the Ig dimeric V domain construct of the invention as replacements for the V domains within the heavy chain and light chain subunits.

In an aspect, the invention provides a nucleotide sequence encoding the V domain construct or the monoclonal full-length immunoglobulin-like molecule of the invention.

In an aspect, the invention provides a pharmaceutical composition comprising the V domain construct and/or the monoclonal full-length immunoglobulin-like molecule of the invention.

In an aspect, the invention provides the V domain construct and/or the monoclonal full-length immunoglobulin-like molecule of the invention for use as a medicament.

### DESCRIPTION OF THE DRAWINGS

For a more complete understanding of the features and advantages of the present invention, reference is now made to the detailed description of the invention along with the accompanying figures and in which:

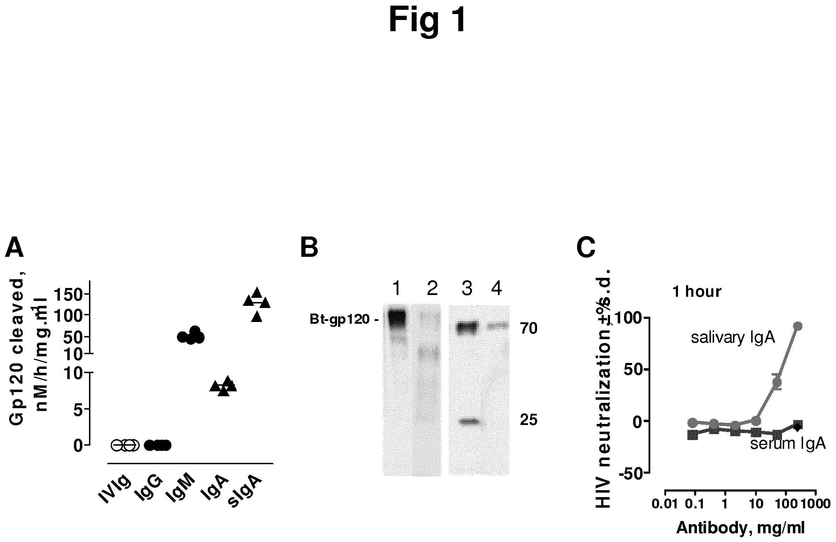

**Fig 1****. (*A*) gp120 hydrolysis by IgAs from HIV negative subjects.** Plot shows proteolytic activity per unit time and per unit Ig mass. Shown are data from serum Igs and salivary IgA (sIgA) from 4 humans and 4 commercial IVIG preparations. (***B***) **Streptavidin-peroxidase stained reducing SDS-gels showing biotinylated gp120 treated with diluent (lane 1) or salivary IgA (lane 2).** Lanes 3 and 4 show, respectively, covalent adducts of E-421-433 and E-hapten formed by a monoclonal human IgM, code 1801. **(C) Neutralizing potency of IgA and IgG Abs purified from pooled serum or saliva.** HIV-1 strain, 97ZA009; host cells, PBMCs. Values are relative to p24 concentrations in test cultures receiving diluent instead of Igs. Serum IgA showed neutralizing activity after 24 h incubation with the virus (not shown).

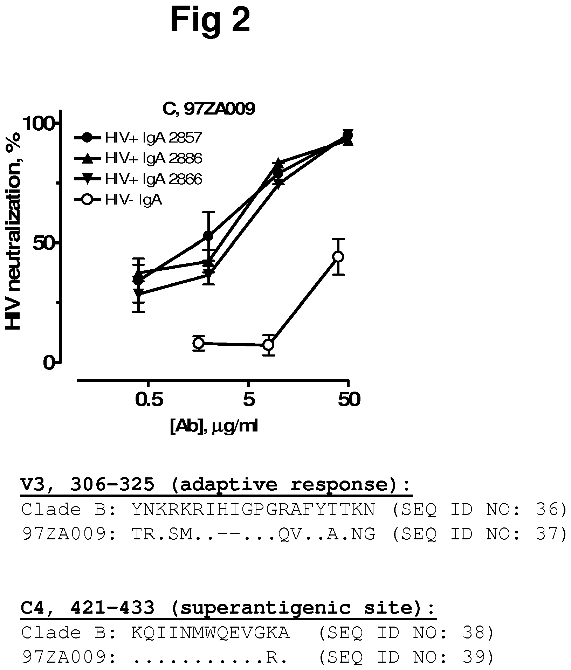

**Fig 2****. Heterologous HIV strain neutralization by serum IgA from presumptive clade B infected S₁₈ subjects. *Top,*** Neutralization of clade C strain ZA009 (R5 coreceptor) by IgA from 3 S₁₈ subjects. IgA incubated 1h with HIV before assaying infectivity using human PBMCs. Means of 4 replicates. ***Bottom,*** Consensus V3 region 306-325 and 421-433 region sequences compared to corresponding strain ZA009 sequences. Dot, identity; dash, gap.

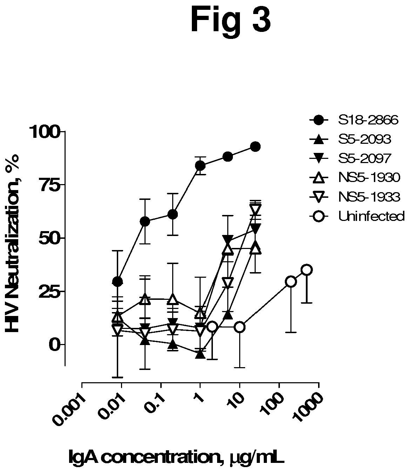

**Fig 3****. Superior neutralizing potency of serum IgA from a S₁₈ subject.** Neutralization of clade C strain ZA009 (R5 coreceptor) by IgA isolated from sera of an uninfected subjects, an S₁₈ subject (i.d. 2866), two S₅ subjects (i.d. 2093 and 2097) and two NS₅ subjects (i.d. 1930 and 1933). HIV (100 TCID₅₀) was incubated with IgA for 1h, then allowed to infect phytohemagglutinin-stimulated human PBMCs. Values are expressed as percent reduction of p24 concentrations in test cultures compared to cultures that received diluent instead of IgA (means ± s.d. of 4 replicates).

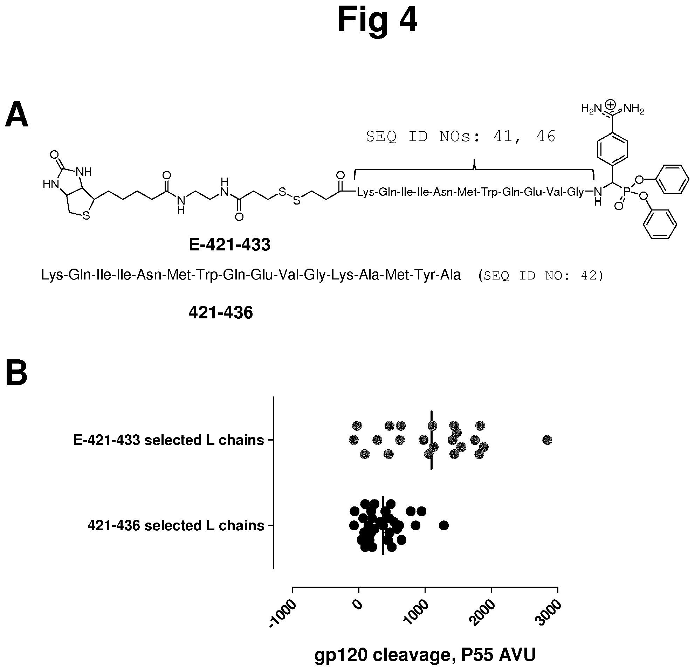

**Fig 4****. Isolation of nucleophilic antibody L chain subunits that recognize gp120 residues 421-433 from a phage-displayed human L chain library. *(A)*** E421-433 and 421-436 structures. **(*B*)** gp120 hydrolyzing activity of L chains selected using E-421-433 or 421-436. For selection methods, see text. The activity was measured using biotinylated gp120 (100 nM) as substrate and expressed as the intensity of the 55kD product band intensity (P55; in arbitrary volume unit, AVU) determined by densitometry of streptavidin-peroxidase stained blots of SDS-electrophoresis gels.

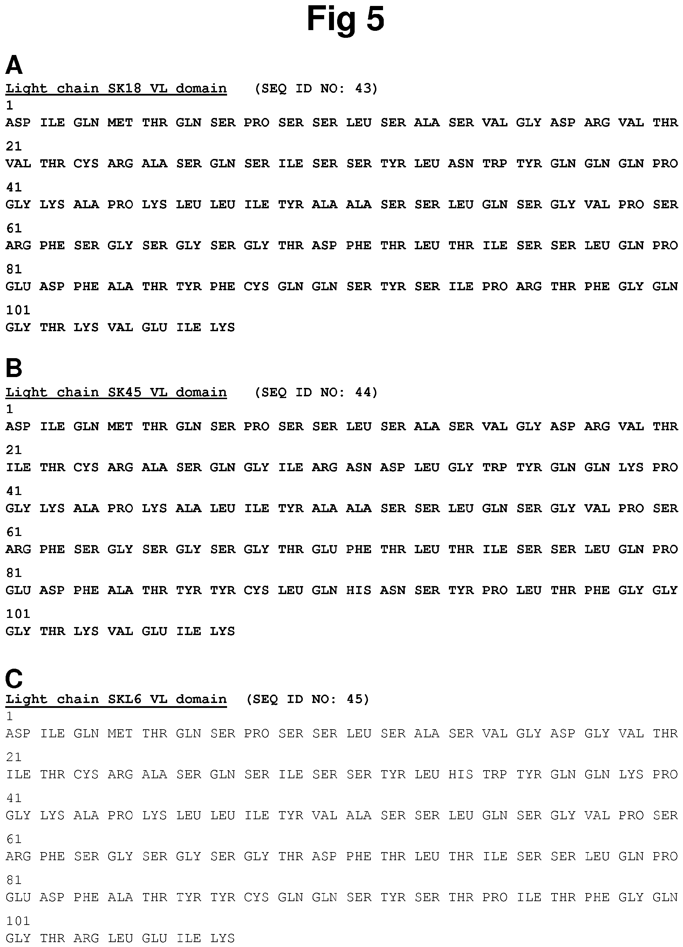

**Fig 5****. Amino acid sequences of VL domains of L chains SK18, SK45 and SKL6.**

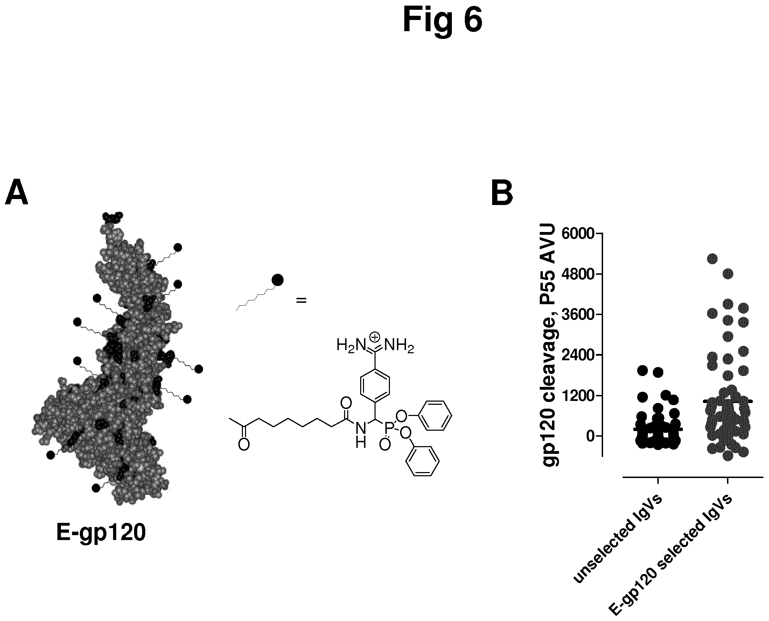

**Fig 6****. Isolation of nucleophilic antibody IgVs by covalent phage selection with E-gp120.** (A) E-gp120 structure. ***(B)*** gp120 hydrolyzing activity of IgVs. For selection methods, see text. The activity was measured using biotinylated gp120 (100 nM) as substrate and expressed as the 55 kD product band intensity (P55; in arbitrary volume unit, AVU) determined by densitometry of streptavidin-peroxidase stained blots of SDS-electrophoresis gels.

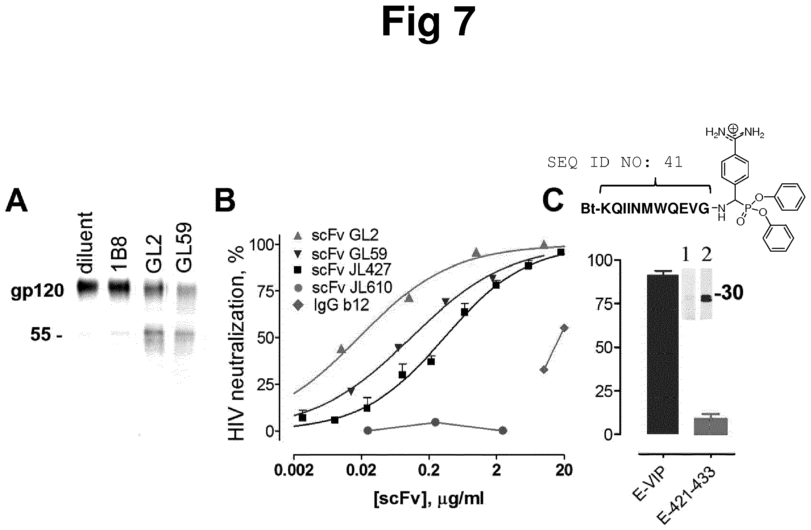

**Fig 7****. Catalytic and neutralizing activities of lupus scFv-*t* clones.** (A) Streptavidin-peroxidase stained blots of SDS-gels showing cleavage of Bt-gp120 (0.1 µM) by purified scFv-*t* clones GL2 and GL59 (20 µg/ml). scFv-*t* 1B8 analyzed in parallel is devoid of catalytic activity. **(*B*)** HIV neutralization (strain ZA009) by scFv-*t* clones. Host cells: PBMCs. **(*C*)** Inhibition of scFv-*t* neutralizing activity by E-421-433. scFv-*t* JL427 (0.24 µg/ml) preincubated for 24 h with E-421-433 (100 µM, see structure in inset) or the control electrophilic peptide E-VIP before assay of neutralizing activity and PBMC. *Inset,* streptavidin stained blot of SDS-gel showing formation of scFv-*t* JL427 adducts with E-421-433 (lane 2; 30 kD) and absence of adducts with E-VIP (lane 1).

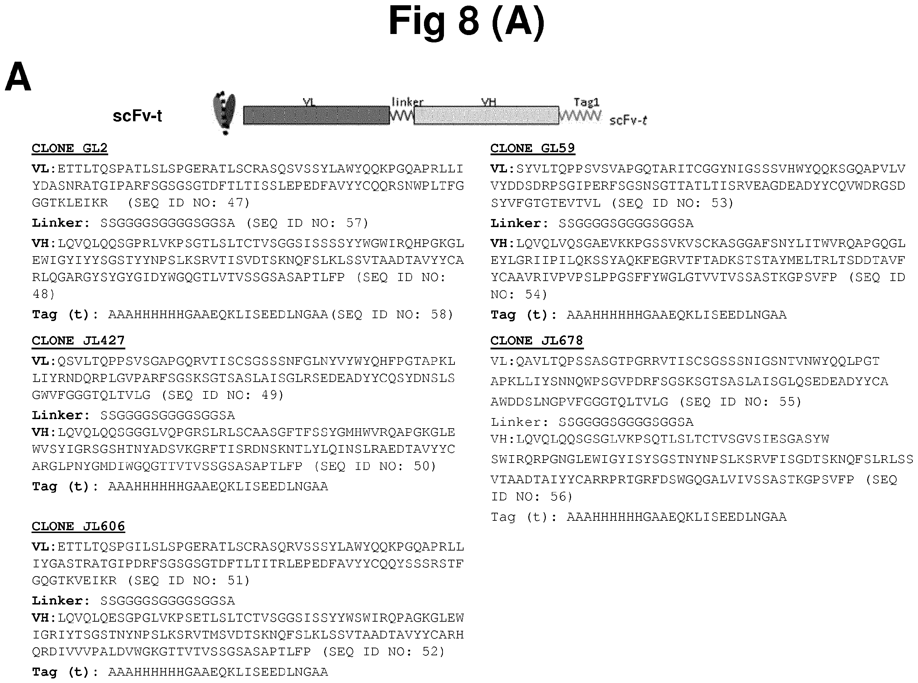

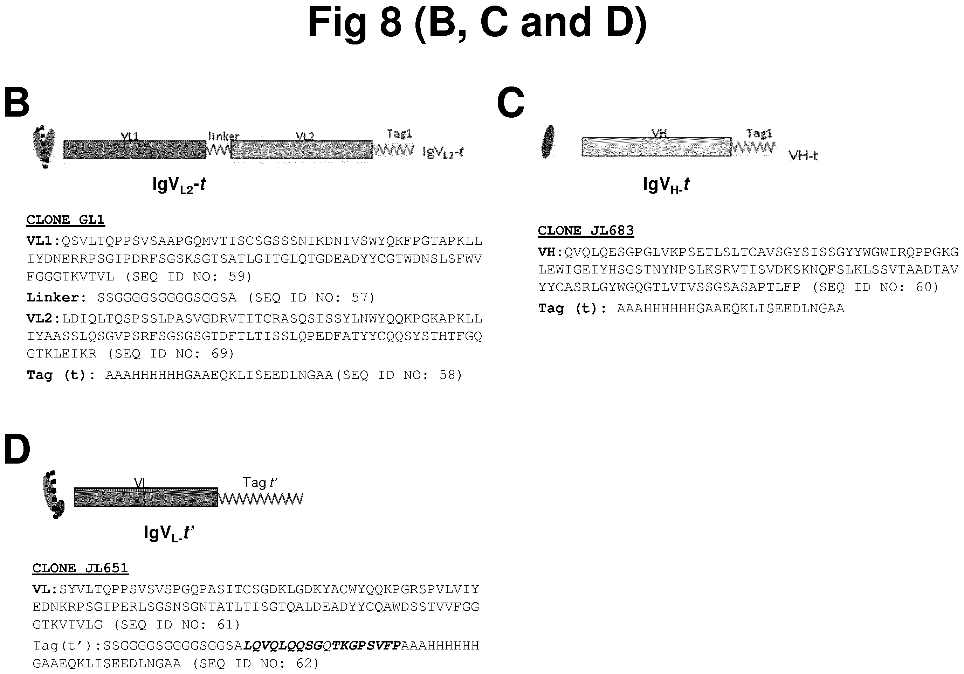

**Fig 8****. Schematic representations of the structures** and **amino acid sequences of** IgVs. **(*A*)** scFv-*t* clones GL2, GL59, JL427, JL606 and JL678. **(*B*)** IgV_{L2}-*t* clone GL1. **(C)** IgV_{H}-*t* clone JL683. **(D)** IgVL-*t*' clone JL651. In JL651, tag *t*' corresponds to the linker, an aberrant polypeptide in place of the VH domain at the C terminus of the linker and the His6-c-myc tag.

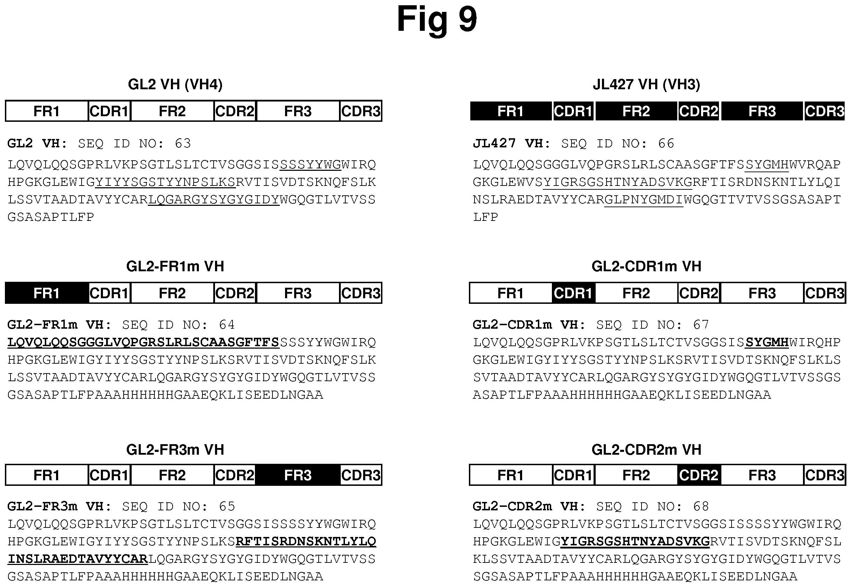

**Fig 9****. Schematic representations of the VH domain structures and amino acid sequences of FR-swapped mutants of scFv-*t* GL2.** Black and white boxes, respectively, are regions derived from scFv-t JL427 and scFv-t GL2. All mutants tested contained the VL domain of scFv-*t* GL2.

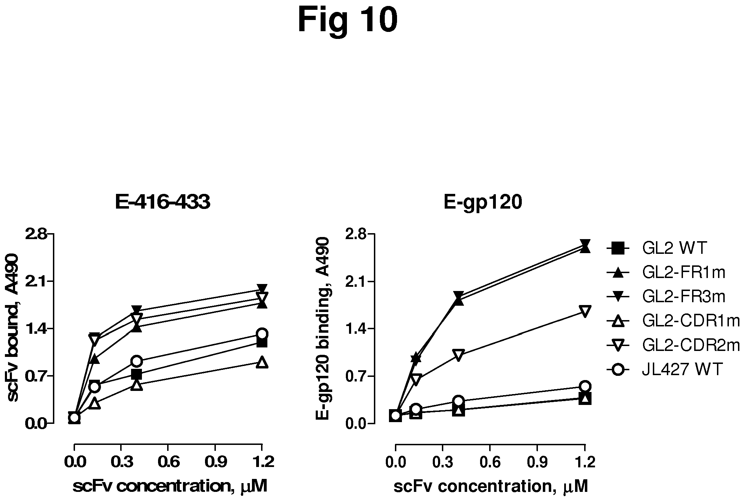

**Fig 10****. E-416-433 and E-gp120 binding by scFv GL2 mutants.** Shown are ELISA data with plates coated with E-416-433 (KLH conjugate; 70 ng peptide equiv/well) or E-gp120 (100 ng/well). Bound scFv was detected with anti-c-myc antibodies and peroxidase-conjugated anti-mouse IgG (Fc specific).

### DETAILED DESCRIPTION OF THE INVENTION

The invention is defined in the appended claims and any other aspects or embodiments set forth herein are for information only.

The term "a" or "an", when used in conjunction with the term "comprising" in the claims and/or the specification, may refer to "one", but it is also consistent with the meaning of "one or more", "at least one", and "one or more than one". Some embodiments of the invention may consist of or consist essentially of one or more elements, method steps, and/or methods of the invention. It is contemplated that any compound, composition, or method described herein can be implemented with respect to any other device, compound, composition, or method described herein.

The term "or" in the claims refers to "and/or" unless explicitly indicated to refer to alternatives only or the alternatives are mutually exclusive, although the disclosure supports a definition that refers to only alternatives and "and/or".

The term "Ig" in the claims refers to any immunoglobulin of the IgM, IgG and classes.

Term "IgV" in the claims refers to any variable domain of the light cand heavy chain Ig subunits with and without incorporation of additional sequences at the termini of the V domains.

The term "tag" in the claims refers to any polypeptide or non-peptide incorporated at the terminus or within the V domain or combination of V domains.

The term "single domain IgV" refers to a V domain that can fulfill the antigen recognition function in the absence of a second full-length V domain.

The term "two domain IgV" in the claims refers to a combination of two V domains that can recognize the antigen.

The term "antigen recognition" refers to the ability to bind the antigen by noncovalent means or covalent means or the ability to catalyze the chemical transformation of the antigen.

In one embodiment of the present disclosure, there are provided classes of IgVs with high level catalytic and binding activities and the desired bioactivity profiles. The structure and properties of the IgVs are exemplified by their reactivities with various antigens of medical interest. Optionally, the IgVs can be isolated from humans with the autoimmune disease systemic lupus erythematosus. Other suitable sources of the IgVs are humans without disease and humans with Alzheimer disease, *S. aureus* infection, HIV infection or HCV infection. For example, humans with Alzheimer disease produce Igs with specificity for Aβ peptide. Consequently, such humans are suitable for isolating IgVs directed to Aβ.

The description discloses several IgVs that catalyze the hydrolysis of Aβ isolated from a library of IgVs derived from lupus patients. The library consists mostly of two domain IgVs containing paired VL-VH antigen combining sites. However, a minority of clones are structurally aberrant because of imprecision of cloning methods used for generating the library. The aberrant structures include IgVs containing two VL domains (designated IgVL2-*t* constructs wherein *t* denotes a short peptide tag included in the IgVs to help identify and purify the recombinant proteins) and IgVs with a single VL domain linked to non-natural polypeptides at the C terminus, e.g., short VH domain sequences containing large internal deletions (designated IgVL-*t*' wherein *t*' denotes the tag region encompassing the aberrant structure at the terminus the VL domain). Random screening methods along with the use of an electrophilic Aβ analog to isolate IgVs with greatest nucleophilic reactivity permitted isolation of catalytic IgVs. Unexpectedly, IgVs with rare IgVL2-*t* and IgVL-*t*' structures expressed the greatest catalytic activity directed to Aβ. The description also discloses single chain Fv constructs (scFvs) obtained by repairing the aberrant VH domain contained in the IgVL-*t*' construct. Such repaired scFvs displayed reduced catalytic activity, indicating that the VH domain generally suppresses VL domain catalytic activity.

The use of electrophilic antigen analogs in the present disclosure is based on the principle that nucleophilic sites located in the V domains imparts to some Igs the ability to catalyze chemical reactions. The nucleophilicity derives from activation of certain amino acid side chains. In serine proteases, precise spatial positioning of the Ser-His-Asp triad allows formation of a hydrogen bonded network that imparts nucleophilic reactivity to the Ser oxygen. Similar sites are present in catalytic V domains [1]. Previous studies have shown that V domain nucleophilic sites bind covalently to phosphonate esters incorporated within antigenic epitopes [2,3].

The electrophilic antigen analogs are designed based on the split site model of catalytic Igs, in which the Ig paratope and nucleophilic sites are treated as two distinct subsites. The analogs are derivatives of polypeptides in which one or more amino acid side chains are linked to the electrophilic phosphonate group as described in US Patent application 20070105092. Examples of other suitable electrophiles are the carbon atom in carbonyl esters, carbonyl amides, carbonates, aldehydes, ketones and aliphatic and aromatic carbonyl compounds; the boron atom in boronates and the vanadium atom in vanadates. Electron withdrawing and donating groups are linked directly to the electrophilic atom or via spacer groups to enhance and decrease the covalent reactivity with IgV nucleophiles. Optionally, a positive charge or a negative charge is placed in the vicinity of the electrophilic atom to mimic the basic residue and acidic residue specificity of catalytic Igs.

The catalytic sites of the IgVs disclosed in the present disclosure can be produced as a result of innate as well as adaptive immune processes. Previous studies have indicated that Ig nucleophilic and proteolytic activities are heritable traits, encoded by germline V domains [4]. Because the catalytic activity is germline-encoded, in principle, the immune system is capable of mounting catalytic Ig V domains directed to any polypeptide antigen. Adaptive specialization for recognition and cleavage of the polypeptide antigen can occur by processes such as V-D-J/V-J junctional diversification and somatic hypermutation of the V domains.

In another embodiment, the description discloses improved catalytic mutants of an IgVL2_{-*t*} obtained by random mutagenesis of the VL domains, display of the mutant proteins on phage surface and isolated mutant IgV_{L2-*t*} clones by covalent phage selection using the electrophilic Aβ analog.

Additional embodiments of the description disclose IgVs directed to proteins belonging to infectious microbes. One such embodiment is isolation of catalytic IgVs to *S. aureus* virulence factors, for example, the virulence factor Efb, obtained by random screening of the lupus IgV library described above. A catalytic scFv and an IgVL2_{-*t*} that hydrolyzed Efb was identified by the by electrophoresis screening methods. Catalytic IgVs with improved catalytic activity can readily be obtained by conducting phage selection procedures as described, for example, using electrophilic analogs of Efb.

The invention discloses the composition of catalytic IgVs directed to the HIV coat protein gp120. These IgVs can be obtained from human IgV libraries as described above. Selection of the IgVs displayed on phage surface can be accomplished using gp120, an electrophilic analog of gp120 or peptides corresponding to the antigenic epitope recognized by the IgVs. HIV gp120 is a B cell superantigen recognized by IgVs from humans without infection. The invention discloses IgVs that recognize the superantigenic peptide epitope composed of gp120 amino acid residues 421-433 or 416-433 and neutralize HIV infection of lymphocytes. Certain IgVs that catalyze the hydrolysis of gp120 are described. As the IgVs neutralize diverse HIV strains they can be applied for immunotherapy of HIV infection. In addition, they can be used as topical microbicides for prevention of heterosexual HIV transmission.

Another embodiment of the invention concerns recognition of B cell superantigens by the V domains, which is thought to occur mainly at conserved V domain residues. IgVs obtained by mutagenesis methods with improved recognition of the HIV gp120 superantigenic site are disclosed. The approach for obtaining the mutants consists of replacing entire FRs or CDRs of the VH domains by corresponding FRs or CDRs drawn from other V domains. In addition, the ability of the VL domain to recognize the HIV gp120 superantigenic site is disclosed. Improved superantigen recognition can also be readily attained by pairing of two V domains that can independently recognize the superantigenic site. Similarly, mutations introduced at individual amino acids in the FRs or CDRs can be employed to improve IgV recognition of the superantigens. Also disclosed are the novel B cell superantigenic properties of the *S. aureus* virulence factors, Map19, ClfA, LukF and SdrE. The disclosed IgV engineering methods can readily be applied to obtain improved recognition of the *S. aureus* virulence factors.

The IgVs can be engineered further to improve their stability in vascular circulation and other anatomic sites, for example by linkage to a polyethylene glycol molecule or the Fc region of Igs. Also disclosed are novel full-length catalytic IgGL and IgML molecules containing antigen combining sites composed of 2 VL domains instead of one VL domain and one VH domain. Also disclosed are two novel bivalent Fc-containing derivatives of an IgV_{L2-*t*}: ***(a)*** an IgGL construct in which one of the V_{L} domain was cloned into the light chain subunit (κ) and the second V_{L} domain into the heavy chain subunit (in place of the V_{H} domain); and ***(b)*** an Fcγ-(IgV_{L2})₂ construct containing two IgV_{L2} components attached to the N terminii of the Fc fragment. The novel IgGL and Fcγ-(IgV_{L2})₂ expressed catalytic activity, indicating that the integrity of catalytic site is unaffected by inclusion of the Fc fragment. Such engineering strategies can be used to prepare long-lived Ig catalysts as immunotherapeutic agents.

The description discloses that variations in the structure of the terminal *t*' region that do not influence of the single domain IgVL-*t*' function. The *t*' region interferes with noncovalent V domain association and maintains the IgVL-*t*' in monomeric form with high level catalytic activity. Optionally, the *t*' region can contain peptide or non-peptide structures that direct the IgV to the desired target anatomic site. For example, inclusion of the polyanionic compound putrescine or the TAT peptide in the *t*' region can facilitate passage of the IgV across the blood-brain-barrier. Similarly, the *t*' region can include an scFv directed to an antigen expressed at the blood-brain-barrier to facilitate transport across the blood-rain-barrier, for example an scFv to the insulin receptor known in the art to transport drugs across the barrier.

Another aspect of the present disclosure consists of identifying polyclonal Igs from blood and mucosal secretions with enriched catalytic activity. This can be accomplished by screening Ig preparations from individual human donors and identifying those immunoglobulin preparations that express catalytic activity greater than the average catalytic activity of Ig preparations. Optionally, the Igs can be obtained from human blood. Alternatively, the present invention also discloses IgG, IgA and IgM preparations obtained from mucosal fluids such as saliva. The catalytic activity is directed to one or more protein, e.g., an *S. aureus* virulence factor, the HCV coat protein E2, the HIV coat protein gp120 or Aβ. For example, the description discloses findings of exceptionally potent HIV neutralization by IgA preparations from long-term survivors of HIV infection. Such Ig preparations are a suitable source of pooled Igs for treatment and prevention of HIV infection. Similarly, screening of human donors has revealed variable levels of catalytic Igs directed to *S aureus* virulence factors protein A, Map19, ClfA, LukF and SdrE. Screening of human donors with HCV infection has revealed enhanced levels of catalytic IgMs to the HCV coat protein E2, with activity levels varying widely from one donor to another. Screening of human donors has revealed similar variations in catalytic IgMs directed to Aβ. The present disclosure conceives pooled Igs from donors expressing enriched levels of catalytic activities to the appropriate antigens to be useful in various medical treatments, including treatment of antibiotic-resistant *S. aureus infection,* HCV infection and Alzheimer disease.

To one skilled in the art, it is evident that protective monoclonal Abs can readily be cloned from the catalytic Ig producing subjects by procedures such as lymphocyte immortalization by Epstein-Barr virus or antibody repertoire cloning and phage display by molecular biology methods. Similarly, the present invention conceives preparation of IgVs from the catalytic Ig producing subjects.

The monoclonal and recombinant Igs disclosed in the present invention can be readily improved by various protein engineering methods familiar to persons skilled in the art. Examples of the engineering techniques are as follows.

The IgVs can be recloned as full-length IgG, IgM and IgA to provide for increased half-life in vivo and increased avidity of protein recognition. When administered to animals, Fv constructs display half-lives in blood on the order of hours. In comparison, the half-life of full-length Abs in blood can be as large as 2-3 weeks. Therefore, to achieve persistent neutralization of the antigen, the preferred reagents are the full-length Igs. On the other hand, the smaller IgV constructs may offer tissue penetration capabilities superior to full-length Igs.

Recloning monovalent IgVs as IgG, IgM and IgA provides for increased antigen binding valencies, respectively. This is useful in some instances. Multivalent binding improves the apparent antigen binding strength, known in the art by the term avidity. In addition, the constant domains imparts important effector functions to Igs, for example, the ability to fix complement, mediate Ig-dependent cellular cytotoxicity and bind Fc receptors. Full-length Igs are readily obtained from IgVs by cloning the V domains into appropriate mammalian cell expression vectors. The vectors contain cDNA encoding the constant domains of the desired Ig class and subclass. The vectors are available commercially, for example, from Lonza. The vectors contain human Ig constant domains flanked by restriction sites for insertion of foreign V domains.

Increased avidity of antigen recognition can also be obtained by forming IgV multimers. For example, tetravalent antibody fragments are generated by placing a 33-amino acid self-aggregating peptide derived from the GNC4 protein at the C terminus of an scFv construct. The peptide associates noncovalently into a 4-helix bundle, permitting expression of multiple valencies.

The sequences of VL, VH and linker domains can be varied by mutagenesis to improve their biological activity. The mutants expressed on the surface of a display vector as described above are allowed to bind the target antigen. This allows separation of the mutants with the highest antigen recognition capability, which in turn can be anticipated to result in improved neutralization capacity. Mutagenesis of the linker peptide that joins the VL and VH domains is designed to improve the interfacial contacts of the VL and VH domains, which allows these domains to form superior antigen binding cavities. To obtain IgVs with improved catalytic activity, mutations are introduced into the FRs or CDRs using mutagenic primers, the mutant molecules are expressed on the surface of phages, and the phages are allowed to bind covalently to the electrophilic antigen analogs. The process is repeated several times, with additional mutations introduced at each cycle followed by the phage separation by antigen binding.

In addition to the strategy described above, favorable mutations can also be introduced in the V domains on a rational basis to improve the catalytic activity. For instance, candidate amino acids suitable for mutagenesis can be identified by molecular modeling or X-ray crystallography information. The ligand can be positioned in the hypothetical binding site to identify candidate residues suitable for rational mutagenesis. For instance, replacement of a small neutral amino acid with a similarly sized charged residue can be attempted as a means to introduce an additional electrostatic stabilizing interaction.

The catalytic VL domain can be paired with the VH domain of other Igs with specific target antigen binding activity. This can improve the binding strength to the target antigen and also result in changes in epitope specificity that can improve antigen neutralizing activity.

The catalytic Igs described herein are generally administered to a patient as a pharmaceutical preparation. The pharmaceutical preparations of the invention are conveniently formulated for administration with a acceptable medium such as water, buffered saline, ethanol, polyol (for example, glycerol, propylene glycol, liquid polyethylene glycol and the like), dimethyl sulfoxide (DMSO), oils, detergents, suspending agents or suitable mixtures thereof. The concentration of the Abs in the chosen medium will depend on the hydrophobic or hydrophilic nature of the medium, as well as the other properties of the catalytic antibodies. Solubility limits may be easily determined by one skilled in the art.

As used herein, "biologically acceptable medium" includes any and all solvents, dispersion media and the like which may be appropriate for the desired route of administration of the pharmaceutical preparation, as exemplified in the preceding paragraph. The use of such media for pharmaceutically active substances is known in the art. Except insofar as any conventional media or agent is incompatible with the catalytic antibody to be administered, its use in the pharmaceutical preparation is contemplated.

In one preferred embodiment, the catalytic Igs can be infused intravenously into the patient. For treatment of certain medical disorders, steps must be taken to ensure that sufficient amounts of the molecules reach their target cells to exert a biological effect. The lipophilicity of the molecules, or the pharmaceutical preparation in which they are delivered may have to be increased so that the molecules can arrive at their target locations. Furthermore, the Igs of the invention may have to be delivered in a cell-targeted carrier so that sufficient numbers of molecules will reach the target cells. Methods for increasing the lipophilicity and targeting of therapeutic molecules, which include capsulation of the Igs of the invention into liposomes, are known in the art.

The IgVs and Igs of the present invention can also be used as topical microbicides, for example as a vaginal cream, foam or film.

The pharmaceutical IgV or Ig preparation is formulated in dosage unit form for ease of administration and uniformity of dosage. Dosage unit form, as used herein, refers to a physically discrete unit of the pharmaceutical preparation appropriate for the patient undergoing treatment. Each dosage should contain a quantity of active ingredient calculated to produce the desired effect in association with the selected pharmaceutical carrier. Procedures for determining the appropriate dosage unit are well known to those skilled in the art. For example, the half-life of syngeneic IgG in the human is about 20 days. Over this period, 60,480 antigen molecules will be cleaved by one molecule of an antibody with a turnover of 2.1/min. It can be seen, therefore, that the peptidase antibodies can express considerably more potent antigen neutralizing activity than stoichiometric, reversibly-binding molecules.

The pharmaceutical preparation comprising the catalytic Igs may be administered at appropriate intervals, for example, twice a week until the pathological symptoms are reduced or alleviated, after which the dosage may be reduced to a maintenance level. The appropriate interval in a particular case would normally depend on the condition and the pathogenic state sought to be treated in the patient.

Standard criteria for acceptable prophylactic or therapeutic agents are employed as follows: (1) Discussions of how such criteria are established for the acceptability of prophylactic or therapeutic agents are common in the art can can be found in such texts as Guide to Clinical Trials by Bert Spilker, Raven Press, New York, 1991. Acceptable criteria for demonstration of efficacy include, for example, measuring clearance of bacterial infection. Conventional monoclonal Igs that act to inhibit the function of particular target molecules are among the most common type of therapeutic agent under development for clinical use by biotechnology and pharmaceutical companies. Accordingly, methods of administration of monoclonal Igs are well known to clinicians of ordinary skill in the art. The Igs contemplated in the present invention will constitute a major improvement over such conventional monoclonal Igs because of their superior potency, resulting in dramatic decrease in the cost of treatment.

### References

1. Gao QS, Sun M, Rees AR, Paul S: Site-directed mutagenesis of proteolytic antibody light chain. J Mol Biol 1995, 253:658-664.
2. Planque S, Taguchi H, Burr G, Bhatia G, Karle S, Zhou YX, Nishiyama Y, Paul S: Broadly distributed chemical reactivity of natural antibodies expressed in coordination with specific antigen binding activity. J Biol Chem 2003, 278:20436-20443.
3. Nishiyama Y, Bhatia G, Bangale Y, Planque S, Mitsuda Y, Taguchi H, Karle S, Paul S: Toward selective covalent inactivation of pathogenic antibodies: a phosphate diester analog of vasoactive intestinal peptide that inactivates catalytic autoantibodies. J Biol Chem 2004, 279:7877-7883.
4. Gololobov G, Sun M, Paul S: Innate antibody catalysis. Mol Immunol 1999, 36:1215-1222.

### EXAMPLE: Catalytic and neutralizing Igs to HIV gp120

***Neutralizing Igs from non-infected humans.*** Over 33 million humans are infected with HIV. No effective vaccine for HIV is available. Progression to AIDS occurs at variable rates in infected subjects. Without treatment, ∼50% of infected subjects die in 10 years [1]. There is consensus that the variable rates of disease progression derive at least in part from discrete immunological factors. Innate and adaptive immune responses help protect against the virus (e.g., [2-4]). Knowledge of resistance factors to HIV can help guide improved treatment and development of a preventive vaccine.

Effective control of HIV by the immune system is thwarted by: ***(a)*** rapid sequence diversification of the immunogenic epitopes in its coat protein gp120; and ***(b)*** the lack of robust adaptive responses to conserved epitopes of viral protein important in virus-host cell interactions. The immune system generally fails to produce Igs to conserved gp120 epitopes with sufficient neutralization potency and breadth to control HIV fully. Ig epitope specificity is an important property governing neutralization. Although rare, neutralizing monoclonal antibodies (MAbs) to gp120 and gp41 have been identified. The best known are: MAb b12 directed to a gp120 determinant overlapping the CD4bs [5]; MAb 2G12 to a mannose-dependent gp120 epitope [6,7]; and MAb 2F5 to a gp41 epitope involved in forming the fusogenic gp41 intermediate [8]. These MAbs neutralize many clade B strains and they protect macaques against challenge with clade B SHIV strains (SIV engineered to express HIV *env*) [9-11]. However, they are often ineffective against HIV belonging to other clades, *e.g.,* clade C strains responsible for >50% of all infections [9]. Igs with epitope specificity similar to MAbs b12, 2G12 or 2F5 are usually not detected in chronically infected HIV subjects. Most Igs in infected individuals are directed to epitopes that mutate rapidly or are inaccessible sterically, allowing infection to progress. Often, the Igs recognize the immunodominant, hypermutable epitopes located within V3 residues 306-325 [12,13]. These Igs usually do not neutralize CCR5-dependent strains or strains with divergent V3 sequences.

gp120 contains a B cell superantigen site recognized by preimmune Igs without requirement for adaptive B cell maturation [14]. B cell superantigen recognition is mediated mostly by contacts at Ig V domain FR residues [15,16]. Most conventional Ig-antigen contacts, in contrast, occur at the CDRs. Peptide mapping studies suggest that the gp120 superantigen site is discontinuous determinant, composed of residues 241-250, 341-350 and 421-440 [17].

We observed that IgMs [18] and IgAs [19] from non-infected humans catalyze the hydrolysis of gp120. From initial rate data, the average turnover number (*k*_{cat}) for a polyclonal IgM preparation was 2.8/min. Salivary IgAs, and to a lesser extent, serum IgAs from uninfected humans hydrolyzed gp120 (**Fig 1A****,B**)**.** IgGs did not. Neutralization potency was modest, determined by p24 enzymeimmunoassay using peripheral blood mononuclear host cells. HIV strain ZA009 was neutralized by treatment with salivary IgA for 1 h **(****Fig 1C****).** Neutralization of the virus by treatment with serum IgA was evident only after prolonged incubation (24 h; not shown), and there was little or no neutralization at the 1 h time point [19]. IgG was ineffective at both time points studies. Thus, the neutralizing activity follows the pattern of the catalytic activity: salivary IgA>serum IgA>>serum IgG.

Specificity was evident from lack of hydrolysis of albumin, soluble epidermal growth factor receptor, FVIII C2 domain and Tat. The catalytic reactions were inhibited completely by an electrophilic analog of gp120 residues 421-433 (E-421-433) [18,19]. IgMs and IgAs formed covalent adducts with E-421-433 stable to boiling and SDS (**Fig 1B**). Similarly, viral neutralization was inhibited by E-421-433 [19]. By N-terminal aa sequencing, the major cleavage site was the 432-433 bond in the SAg site [18,19]. Salivary sIgA also hydrolyzed the V83-E84 and Y321-T322 bonds. These observations indicate a nucleophilic catalytic mechanism in which noncovalent 421-433 recognition permits selective gp120 hydrolysis. We and others have previously reported hydrolysis of multiple bonds in polypeptide Ags by Igs, some distant from the binding epitope [20,21]. The observations are consistent with a split-site model involving distinct subsites responsible for noncovalent binding and catalysis [22]. The catalytic subsite fails to make stable contacts with the Ag in the noncovalent immune complex. As the transition state develops, different peptide bonds become positioned in register with the catalytic site. When the Ab recognizes a conformational epitope, the alternate cleavage sites must be spatial neighbors, but they can be distant in the linear sequence, producing a complex fragmentation pattern.

***Neutralizing Igs from long-term survivors of HIV infection.*** Adaptive synthesis of specific Igs to microbial antigens usually entails sequence diversification at the CDRs of the B cell receptor (BCR), guided by selection pressures enabling proliferation of B cells with the highest affinity BCRs. Adaptive improvement of Igs to superantigenic epitopes is thought to be proscribed by B cell down-regulatory signals generated upon superantigen interactions at BCR FRs [23].

We studied IgA preparations from the blood of three subjects who have survived for 18-20 years following infection by presumptive clade B HIV strains despite little or no antiretroviral therapy (designated survivor "S₁₈" subjects; CD4 cell counts 178-426/µl). Serum IgA preparations from the S₁₈ subjects displayed readily detectable gp120 hydrolyzing activity. Identical fragmentation profiles were observed by treating biotinylated gp120 with IgA from HIV infected and non-infected humans. Diverse clade B and heterologous clade C strains were neutralized with exceptional potency by the S₁₈ IgAs (**Fig 2** and **Table 1;** 1 h incubation with virus; n=11 R5-dependent strains). The immunodominant V3 306-325 epitope is highly variable in these strains and the 421-433 epitope is mostly conserved (see example sequences, **Fig 2****).** In comparison, the reference MAb commonly cited as a broad neutralizing Ig, clone b12, did not neutralize many strains, and when neutralization was evident, the potency was substantially lower **(Table 1).** The neutralizing potency of S₁₈ IgAs was about 3 orders of magnitude superior to IgAs from uninfected subjects and IgAs obtained 1-5 years after seroconversion from subjects who succumbed to AIDS (designated non-survivors "NS₅" subjects; CD4⁺ T cell counts <200/µl; n=10) or survived without development of AIDS (S₅ subjects, CD4⁺ T cell counts >200/µl; n=10) (**Fig 3**)**.** gp120 binding by IgAs from S₁₈, NS₅ and S₅ subjects was comparable, indicating similar conventional antibody activity. The restricted presence of potently neutralizing IgAs in very late infection suggests an unconventional adaptive response. In addition to binding to the immunodominant V3 epitope, increased binding of a non-hydrolyzable electrophilic analog of gp120 residues 421-433 by S₁₈ IgAs was evident. These observations suggest that survivors with prolonged HIV infection mount a late adaptive IgA response to gp120 with properties that can slow progression to AIDS.

**Table 1. Heterologous clade C HIV and diverse clade B strain neutralization by IgAs from 3 S₁₈ subjects. Clade C strains: 97ZA009, 98TZ013, 98TZ017, Du123, Du156, Du172, Du422. Clade B strains: ADA, PAVO and QH0692. All CCR5-dependent. PBMC hosts, p24 assays. Dose-dependent HIV neutralization was evident in all assays.**

| | Number of strains neutralized/Number of strains tested (IC₅₀ range; mean ± S.D, µg/ml) | |
|---|---|---|
| | clade B | clade C |
| IgA 2857 | 3/3 (0.08-0.20; 0.12 ± 0.07) | 6/6 (0.01-2.70; 0.9 ± 1.3) |
| IgA 2866 | 3/3 (0.002-1.000; 0.37 ± 0.55) | 7/7 (0.008-1.960; 0.5 ± 1.0) |
| IgA 2886 | 3/3 (0.20-0.80; 0.53 ± 0.33) | 7/7 (0.10-10.70; 2.4 ± 3.8) |
| IgG B12 | 3/4 (0.90-10.00; 3.96 ± 5.22) | 3/8 (4.10-10.50; 6.1 ± 3.5) |

***Ig L chain subunits directed to gp120 residues 421-433.*** Previously, increased polyclonal Igs that bind the 421-436 region were observed in lupus sera [24]. We and others reported increased catalytic Igs in autoimmune diseases. Several reports have noted that HIV infection is infrequent in lupus patients [25-29]. The mechanism of increased Igs to the 421-433 region in lupus is not clear. One possibility is Ig synthesis driven by a homologous antigen. By searching the sequence databases, we found nucleotide sequence homology between the 421-433 region and a human endogenous retroviral sequence (HERV) [30]. HERV expression is increased in lupus [31].

To isolate nucleophilic antibody L chain subunits that recognize gp120 residues 421-433, we prepared a phage-displayed light chain library from lupus patients. The library was fractionated by the following two methods: (a) a two step procedure entailing covalent selection using an electrophilic hapten as described in [32] followed by non-covalent selection using the peptide corresponding to gp120 residues 421-436; and (b) a one step, affinity-driven covalent selection using the electrophilic analog of gp120 421-433 containing the phosphonate group at the C-terminus (E-421-433; **Fig 4A**)**.** Procedures for preparation of the phage light chain library and E-421-433 were described previously [32,33]. Selection procedures were essentially as in [32] and [30]. For example, E-421-433 complexed phages were captured with streptavidin-agarose, the noncovalently bound phages were removed by acid washing (pH 2.7), and covalently bound phages were eluted by 2-mercaptoethanol reduction of the S-S group in the E-421-433 linker. Covalently selected light chains were expressed in soluble form and purified by metal affinity chromatography.

Thirty one L chains selected using the 421-436 peptide and 22 L chains selected using E-421-433 were studied for gp120 hydrolyzing activity. The proteolysis assay was conducted using biotinylated gp120 (Bt-gp120; prepared from recombinant gp120 of MN strain; baculovirus expression system) as substrate followed by SDS-electrophoresis and densitometry of streptavidin-peroxidase stained blots. The group of L chains selected using E-421-433 displayed significantly greater proteolytic activity than the 421-436 selected L chains (**Fig 4B****;** mean ± SE values, respectively, 1098 ± 157 vs 369 ± 56 AVU; P<0.0001, unpaired two-tailed t-test). This result indicates that, although both groups contain 421-433-directed nucleophilic antibody fragments, the E-421-433 selection can afford more efficient enrichment of the L chains compared to the two-step selection.

Two L chains each from the 421-436 selected group (clones SK18 and SK45) and the E-421-433 selected group (clones SK2C2 and SK2F5) were assessed for their ability to neutralize a clade C HIV primary isolate (97ZA009) using peripheral blood mononuclear cells (PBMCs) as hosts. All four light chains neutralized this HIV isolate (IC₅₀/IC₈₀ values: SK18, 0.6/2.1; SK45, 0.6/3.9; SK2C2, <0.01/0.13; SK2F5, <0.01/0.07 µg/mL).

The cDNAs encoding the 421-436-selected L chains SK18 and SK45 and E-421-433 selected L chain SKL6 were sequenced. Their deduced V_{L} domain amino acid sequences are shown in **Fig 5****.**

***IgV fragments isolated by covalent phage selection with gp120 and E-gp120.*** To isolate nucleophilic IgV fragments, the phage-displayed IgV library derived from lupus patients was fractionated for binding to gp120 or E-gp120 **(****Fig 6A****).** Procedures for preparation of the phage IgV library and E-gp120 were described previously [32,34]. As described in EXAMPLE 1, a majority of the clones in this library are scFv constructs mimicking the physiological structures of Ig combining sites. The remaining clones are IgVs with unnatural structures, including IgV_{L2-*t*} and IgV_{L-*t*}' structures. IgV-displaying phages were packaged from the library and subjected to noncovalent selection by affinity chromatography on recombinant gp120 (immobilized on Biorad Affigel-10). Phages from the acid eluate were then subjected to covalent selection with biotinylated E-gp120. E-gp120 complexed phages were captured with immobilized anti-biotin IgG, and following extensive washing with a neutral buffer, bound phages were allowed to elute using the pH 2.7 buffer. The gp120 selected and E-gp120 selected IgVs were expressed in soluble form and purified by metal affinity chromatography.

Sixty two IgVs obtained by E-gp120 selection were assayed for gp120 hydrolysis activity along with a random collection of 61 IgVs from the source library obtained without selection. The proteolysis assay was conducted using biotinylated gp120 as in the preceding section. The E-gp120 selected group displayed significantly greater proteolytic activity than the unselected group (**Fig 6B****;** mean ± SE intensity of 55kD product band generated by the E-gp120 selected group vs unselected group, 1027 ± 167 vs 196 ± 58 AVU; P<0.0001, unpaired two-tailed t-test). Examples of gp120 hydrolyzing IgVs obtained by E-gp120 selection (clones GL2 and GL59) are shown in **Fig 7A****.**

About 50% of the IgV clones selected using gp120 and 100% of the clones selected using E-gp120 neutralized the clade C strain ZA009 using PBMC hosts (**Table 2**)**.** The neutralizing potency of the E-gp120 selected clones was markedly superior to the gp120 selected clones, suggesting the functional advantage gained due to greater nucleophilicity (**Table 2**)**.** The neutralizing potency of example IgV clones GL2, GL59 and JL427 are shown in **Fig 7B****.** scFv 610 in this figure is a control non-neutralizing clone, and IgG b12 is a leading monoclonal IgG commonly cited in the literature as a broadly neutralizing IgG. Neutralization by the IgV clones was inhibited by treatment with E-421-433 but not the irrelevant E-VIP (**Fig 7C**)**.**

**Table 2. HIV neutralization (clade C, R5, ZA009) by lupus scFv fragments selected using gp120 or E-gp120. The characteristics of the lupus library are as in ref [32]. The scFv were purified by Ni-NTA chromatography and tested for neutralization using ZA009 (clade C, R5) as in ref [35].**

| *Selecting Antigen* | # of *clones, tested* | # of *clones, neutralized* | *Potency (EC₅₀, µg*/*ml)* | | |
|---|---|---|---|---|---|
| | | | 0.25-2.5 | 0.025-0.25 | 0.0025-0.025 |
| gp120 | 52 | 25 | 17 | 8 | 0 |
| E-gp120 | 14 | 14 | 0 | 6 | 8 |

By nucleotide sequencing, the E-gp120 selected IgVs GL2 and GL59 were identified as scFv constructs that mimic the structure of physiological Ig combining sites. The gp120 selected IgV clones JL606, JL678 and JL427 were also scFvs. As in the E-Aβ selection studies (EXAMPLE 1), certain selected IgV clones contained aberrant structures. These are the heterodimeric IgV_{L2} clone GL1, the single domain IgV_{H-*t*} clone JL683 without a VL domain partner but with the expected tag at the C terminus, and the single domain IgV_{L}-*t*' clone JL651 containing a VH domain with a large internal deletion. Tag *t* refers to the 27 residue c-myc/his6 sequence at the C terminus, and the designation *t*' is used to denote the unexpected VH peptide with tag *t* at the C terminus in IgV_{L}-*t*' JL651. Schematic representations of the structures and amino acid sequences of these clones are in **Fig 8****.** The HIV neutralizing potency of these clones is shown in **Table 3.**

**Table 3. HIV neutralization potency of E-gp120-selected and gp120-selected IgVs. HIV, 97ZA009; host, PBMC. IC₅₀ values are in ng/mL units, and mean ± SEM are shown for clones tested in multiple independent assays (numbers in parenthesis represents the number of assays).**

| | E-gp120 selected | | | gp120 selected | | | | |
|---|---|---|---|---|---|---|---|---|
| | scFv GL2 | scFv GL59 | IgV_{L2}-*t* GL1 | IgV_{H-t} JL683 | IgV_{L-t'} JL651 | scFv JL606 | scFv JL678 | scFv JL427 |
| IC₅₀, ng/mL | 8.8 ± 5.3 (3) | 2.5 ± 0.9 (6) | 8.1 ± 5.8 (2) | 3.7 | 29.3 | 82.5 | 141.0 | 300 ± 350 (24) |

scFv clones GL2 and JL427 neutralized diverse R5-dependent HIV strains (clades A, B, C; **Table 4**)**.** Another intriguing finding is that scFv clone JL427 neutralized primary HIV strains in the PBMC assay strains but not the corresponding pseudovirions assayed in reporter cell lines. This implies that the 421-433 region may be expressed on native virions and pseudovirions in Ig-recognizable and non-recognizable conformations, respectively. Another Ig that neutralizes primary HIV isolates but not pseudovirions is reported [36]. Importantly, a recent study indicates that neutralization of primary HIV strains by Igs from certain infected subjects correlates with lack of progression whereas neutralization of pseudovirions does not [37]. For these reasons, it is appropriate to rely on infection assays using primary HIV isolates. In these assays, the 421-433 recognizing Igs surpass the potency and breadth of neutralization of other known Igs.

**Table 4: Cross-clade HIV neutralization by lupus scFvs GL2 and JL427. PBMC assays. HIV strains (clade/coreceptor strain names): A/R5 92RW008; A/X4 92USNG17; B/R5 SF162, 92BR014, BAL, PVO, TRO, ADA; B/X4 92HT599, Tybe; B/R5X4 BZ167, 92BR014; C/R5 98BR004, 97ZA009, 98TZ013, 98TZ017, Du123, Du422, Du172;D/R5 92UG266; D/X4 92UG001, 92UG266, 92UG046. Dose dependent HIV neutralization was evident in all cases.**

| HIV clade | Number of strains neutralized/Number of strains tested (IC50 range, mean ± S.D.; µg/ml) | |
|---|---|---|
| | scFv GL2 | scFv JL427 |
| A | 2/2 (0.8-4.2; 2.5 ± 2.4) | 1/1 (0.2; N/A) |
| B | 5/6 (0.007-3.7; 1.5 ± 1.9) | 6/8 (0.03-5.1; 1.3 ± 2.0) |
| C | 5/5 (0.015-2.7; 0.9 ± 1.3) | 6/6 (0.007-4.8; 0.9 ± 1.9) |
| D | 0/3 | 0/4 |

***IgV FR*/*CDR swapping.*** Antigen-specific Igs are usually synthesized adaptively by mutations occurring in the CDRs. In comparison, the superantigenic site of gp120 is thought to be recognized by Igs via contacts at conserved residues located mainly in VH3 domain FR1 and FR3 with additional contributions provided by CDR 1 and CDR2 [15,16]. Conventional Igs that bind the gp120 superantigenic site predominantly utilize VH3 family genes [14], suggesting that conserved VH3 family gene elements preferentially recognize the superantigenic site by noncovalent means. Replacement of the individual FRs and CDRs in a gp120 binding VH3 family Fab by the corresponding FRs and CDRs of a non-VH3 family, non-gp120 binding Fab resulted in loss of the gp120 binding activity of the former Fab [15].

The present invention discloses FR and CDR swapping as a novel means to improve recognition of the gp120 superantigenic site by Igs. The neutralizing scFv GL2 contains a VH4 family VH domain and the neutralizing scFv JL427 contains a VH3 family VH domain. FR1, FR3, CDR1 or CDR2 of the scFv GL2 VH domain were replaced individually by the corresponding regions of the VH3 family scFv JL427. This was done by a PCR-based substitution method using mutagenic primers coding for the aforementioned scFv JL427 regions and scFv GL2 in pHEN2 plasmid as template. PCR products were treated with T4 polynucleotide kinase, *Dpn*I to remove the methylated template DNA, and the purified mutant DNA was circularized by ligation. The DNA was sequenced to confirm the presence of the desired mutations, and mutant scFv mutants were expressed and purified by metal affinity chromatography as described [32]. **Fig 9** shows a schematic representation of the mutants and their sequences. The mutants are designated GL2-FR1m, GL2-FR3m, GL2-CDR1m and GL2-CDR2m, wherein GL2 denotes the scFv GL2 containing FR1, FR3, CDR1 and CDR3 from scFv JL427.

ELISAs using plates coated with E-gp120 or KLH conjugated to E-416-433 were employed to determine recognition of the gp120 superantigenic site. Preparation of E-gp120 and study of its binding by the scFv constructs was as in [34]. Preparation of E-416-433 was as in [38]. This peptide analog displays improved reactivity to Igs that recognize the gp120 superantigenic site compared to E-421-433, a finding consistent with the report that inclusion of the N terminal 416-420 residues induces a conformational change in the 421-433 region [39]. E-416-433 contains phosphonates at Lys422 and Lys432. Its identity was verified by electrospray ionization-mass spectrometry. Conjugation of the peptide analog to KLH was as in [40]. The nonhydrolyzable E-antigen probes were employed (instead of unmodified gp120 and unmodified 416-433 peptide) to preclude possible hydrolysis by scFv GL2, which has the ability to catalyze gp120 degradation.

The mutant scFv constructs GL2-FR1m, GL2-FR3m displayed markedly increased binding to E-gp120 and E-416-433 compared to wildtype (unmutated) scFv GL2 (**Fig 10**)**.** These mutants also displayed increased binding to the two antigenic probes compared to the scFv JL427. scFv JL427 contains the full-length complement of gp120 superantigen binding expressed by a full-length VH3 domain, whereas the mutants contain only small fragments derived from the VH3 domain of JL427. The likely explanation is that the scFv GL2 VL domain provides an important contribution in recognition of the superantigenic epitope of gp120. The obvious alternative route to improving gp120 superantigen site binding, therefore, is to screen and select for optimally paired VL and VH domains from diverse combinatorial libraries of the two domains.

### References in EXAMPLE

1. Time from HIV-1 seroconversion to AIDS and death before widespread use of highly-active antiretroviral therapy: a collaborative re-analysis. Collaborative Group on AIDS Incubation and HIV Survival including the CASCADE EU Concerted Action. Concerted Action on SeroConversion to AIDS and Death in Europe. Lancet 2000, 355:1131-1137.
2. Cecilia D, Kleeberger C, Munoz A, Giorgi JV, Zolla-Pazner S: A longitudinal study of neutralizing antibodies and disease progression in HIV-1-infected subjects. J Infect Dis 1999, 179:1365-1374.
3. Townsley-Fuchs J, Kam L, Fairhurst R, Gange SJ, Goodglick L, Giorgi JV, Sidell N, Detels R, Braun J: Human immunodeficiency virus-1 (HIV-1) gp120 superantigen-binding serum antibodies. A host factor in homosexual HIV-1 transmission. J Clin Invest 1996, 98:1794-1801.
4. Gaudieri S, Nolan D, McKinnon E, Witt CS, Mallal S, Christiansen FT: Associations between KIR epitope combinations expressed by HLA-B/-C haplotypes found in an HIV-1 infected study population may influence NK mediated immune responses. Mol Immunol 2005, 42:557-560.
5. Burton DR, Pyati J, Koduri R, Sharp SJ, Thornton GB, Parren PW, Sawyer LS, Hendry RM, Dunlop N, Nara PL, et al.: Efficient neutralization of primary isolates of HIV-1 by a recombinant human monoclonal antibody. Science 1994, 266:1024-1027.
6. Sanders RW, Venturi M, Schiffner L, Kalyanaraman R, Katinger H, Lloyd KO, Kwong PD, Moore JP: The mannose-dependent epitope for neutralizing antibody 2G12 on human immunodeficiency virus type 1 glycoprotein gp120. J Virol 2002, 76:7293-7305.
7. Scanlan CN, Pantophlet R, Wormald MR, Ollmann Saphire E, Stanfield R, Wilson IA, Katinger H, Dwek RA, Rudd PM, Burton DR: The broadly neutralizing anti-human immunodeficiency virus type 1 antibody 2G12 recognizes a cluster of alphal-->2 mannose residues on the outer face of gp120. J Virol 2002, 76:7306-7321.
8. Ofek G, Tang M, Sambor A, Katinger H, Mascola JR, Wyatt R, Kwong PD: Structure and mechanistic analysis of the anti-human immunodeficiency virus type 1 antibody 2F5 in complex with its gp41 epitope. J Virol 2004, 78:10724-10737.
9. Binley JM, Wrin T, Korber B, Zwick MB, Wang M, Chappey C, Stiegler G, Kunert R, Zolla-Pazner S, Katinger H, et al.: Comprehensive cross-clade neutralization analysis of a panel of anti-human immunodeficiency virus type 1 monoclonal antibodies. J Virol 2004, 78:13232-13252.
10. Mascola JR, Louder MK, VanCott TC, Sapan CV, Lambert JS, Muenz LR, Bunow B, Birx DL, Robb ML: Potent and synergistic neutralization of human immunodeficiency virus (HIV) type 1 primary isolates by hyperimmune anti-HIV immunoglobulin combined with monoclonal antibodies 2F5 and 2G12. J Virol 1997, 71:7198-7206.
11. Veazey RS, Shattock RJ, Pope M, Kirijan JC, Jones J, Hu Q, Ketas T, Marx PA, Klasse PJ, Burton DR, et al.: Prevention of virus transmission to macaque monkeys by a vaginally applied monoclonal antibody to HIV-1 gp120. Nat Med 2003, 9:343-346.
12. Gorny MK, Xu JY, Karwowska S, Buchbinder A, Zolla-Pazner S: Repertoire of neutralizing human monoclonal antibodies specific for the V3 domain of HIV-1 gp120. J Immunol 1993, 150:635-643.
13. Profy AT, Salinas PA, Eckler LI, Dunlop NM, Nara PL, Putney SD: Epitopes recognized by the neutralizing antibodies of an HIV-1-infected individual. J Immunol 1990, 144:4641-4647.
14. Berberian L, Goodglick L, Kipps TJ, Braun J: Immunoglobulin VH3 gene products: natural ligands for HIV gp120. Science 1993, 261:1588-1591.
15. Neshat MN, Goodglick L, Lim K, Braun J: Mapping the B cell superantigen binding site for HIV-1 gp120 on a V(H)3 Ig. Int Immunol 2000, 12:305-312.
16. Karray S, Juompan L, Maroun RC, Isenberg D, Silverman GJ, Zouali M: Structural basis of the gp120 superantigen-binding site on human immunoglobulins. J Immunol 1998, 161:6681-6688.
17. Goodglick L, Zevit N, Neshat MS, Braun J: Mapping the Ig superantigen-binding site of HIV-1 gp120. J Immunol 1995, 155:5151-5159.
18. Paul S, Karle S, Planque S, Taguchi H, Salas M, Nishiyama Y, Handy B, Hunter R, Edmundson A, Hanson C: Naturally occurring proteolytic antibodies: selective immunoglobulin M-catalyzed hydrolysis of HIV gp120. J Biol Chem 2004, 279:39611-39619.
19. Planque S, Mitsuda Y, Taguchi H, Salas M, Morris MK, Nishiyama Y, Kyle R, Okhuysen P, Escobar M, Hunter R, et al.: Characterization of gp120 Hydrolysis by IgA Antibodies from Humans without HIV Infection. AIDS Res Hum Retroviruses 2007, 23:1541-1554.
20. Hifumi E, Mitsuda Y, Ohara K, Uda T: Targeted destruction of the HIV-1 coat protein gp41 by a catalytic antibody light chain. J Immunol Methods 2002, 269:283-298.
21. Sun M, Gao QS, Kirnarskiy L, Rees A, Paul S: Cleavage specificity of a proteolytic antibody light chain and effects of the heavy chain variable domain. J Mol Biol 1997, 271:374-385.
22. Paul S: Natural catalytic antibodies. Mol Biotechnol 1996, 5:197-207.
23. Juompan L, Lambin P, Zouali M: Selective deficit in antibodies specific for the superantigen binding site of gp120 in HIV infection. Faseb J 1998, 12:1473-1480.
24. Bermas BL, Petri M, Berzofsky JA, Waisman A, Shearer GM, Mozes E: Binding of glycoprotein 120 and peptides from the HIV-1 envelope by autoantibodies in mice with experimentally induced systemic lupus erythematosus and in patients with the disease. AIDS Res Hum Retroviruses 1994, 10:1071-1077.
25. Chang BG, Markowitz GS, Seshan SV, Seigle RL, D'Agati VD: Renal manifestations of concurrent systemic lupus erythematosus and HIV infection. Am J Kidney Dis 1999, 33:441-449.
26. Daikh BE, Holyst MM: Lupus-specific autoantibodies in concomitant human immunodeficiency virus and systemic lupus erythematosus: case report and literature review. Semin Arthritis Rheum 2001, 30:418-425.
27. Palacios R, Santos J, Valdivielso P, Marquez M: Human immunodeficiency virus infection and systemic lupus erythematosus. An unusual case and a review of the literature. Lupus 2002, 11:60-63.
28. Sekigawa I, Lee S, Kaneko H, Iida N, Hashimoto H, Hirose S, Kaneko Y: The possible role of interleukin-16 in the low incidence of HIV infection in patients with systemic lupus erythematosus. Lupus 2000, 9:155-156.
29. Wallace DJ: Lupus, acquired immunodeficiency syndrome, and antimalarial agents. Arthritis Rheum 1991, 34:372-373.
30. Nishiyama Y, Karle S, Planque S, Taguchi H, Paul S: Antibodies to the superantigenic site of HIV-1 gp120: hydrolytic and binding activities of the light chain subunit. Mol Immunol 2007, 44:2707-2718.
31. Urnovitz HB, Murphy WH: Human endogenous retroviruses: nature, occurrence, and clinical implications in human disease. Clin Microbiol Rev 1996, 9:72-99.
32. Paul S, Tramontano A, Gololobov G, Zhou YX, Taguchi H, Karle S, Nishiyama Y, Planque S, George S: Phosphonate ester probes for proteolytic antibodies. J Biol Chem 2001, 276:28314-28320.
33. Taguchi H, Burr G, Karle S, Planque S, Zhou YX, Paul S, Nishiyama Y: A mechanism-based probe for gp120-Hydrolyzing antibodies. Bioorg Med Chem Lett 2002, 12:3167-3170.
34. Paul S, Planque S, Zhou YX, Taguchi H, Bhatia G, Karle S, Hanson C, Nishiyama Y: Specific HIV gp120-cleaving antibodies induced by covalently reactive analog of gp120. J Biol Chem 2003, 278:20429-20435.
35. Karle S, Planque S, Nishiyama Y, Taguchi H, Zhou YX, Salas M, Lake D, Thiagarajan P, Arnett F, Hanson CV, et al.: Cross-clade HIV-1 neutralization by an antibody fragment from a lupus phage display library. Aids 2004, 18:329-331.
36. Brown BK, Karasavvas N, Beck Z, Matyas GR, Birx DL, Polonis VR, Alving CR: Monoclonal antibodies to phosphatidylinositol phosphate neutralize human immunodeficiency virus type 1: role of phosphate-binding subsites. J Virol 2007, 81:2087-2091.
37. Brown BK, Wieczorek L, Rosa Borges A, Sanders-Buell E, Horak J, Robb M, Birx D, Michael N, McCutchan F, Polonis V: HIV neutralization is profoundly affected by the cell model system used in vitro when a multiclade virus panel and polyclonal antibodies are employed. AIDS Vaccine 2007. Seattle, WA; Aug 20-23, 2007 Abstract# OA05-08 (http://www.hivvaccineenterprise.org/_dwn/Oral_Sessions.pdf).
38. Nishiyama Y, Bhatia G, Bangale Y, Planque S, Mitsuda Y, Taguchi H, Karle S, Paul S: Toward selective covalent inactivation of pathogenic antibodies: a phosphate diester analog of vasoactive intestinal peptide that inactivates catalytic autoantibodies. J Biol Chem 2004, 279:7877-7883.
39. Reed J, Kinzel V: Primary structure elements responsible for the conformational switch in the envelope glycoprotein gp120 from human immunodeficiency virus type 1: LPCR is a motif governing folding. Proc Natl Acad Sci U S A 1993, 90:6761-6765.
40. Karle S, Nishiyama Y, Taguchi H, Zhou YX, Luo J, Planque S, Hanson C, Paul S: Carrier-dependent specificity of antibodies to a conserved peptide determinant of gp120. Vaccine 2003, 21:1213-1218.

## Claims

1. An isolated or purified variable (V) domain construct, which catalyzes the hydrolysis of HIV gp120, and which comprises two individual immunoglobulin (Ig) V domains, wherein said construct comprises:
- the light chain V (VL) domain GL2-VL having the amino acid sequence: 
ETTLTQSPATLSLSPGERATLSCRASQSVSSYLAWYQQKPGQAPRLLIYDASNR
ATGIPARFSGSGSGTDFTLTISSLEPEDFAVYYCQQRSNWPLTFGGGTKLEIKR
(SEQ ID NO: 47); and
- and an Ig heavy chain V (VH) domain comprising a sequence selected from the group consisting of:
GL2-FRlm having the amino acid sequence: 
LQVQLQQSGGGLVQPGRSLRLSCAASGFTFSSSSYYWGWIRQHPGKGLEWI
GYIYYSGSTYYNPSLKSRVTISVDTSKNQFSLKLSSVTAADTAVYYCARLQG
ARGYSYGYGIDYWGQGTLVTVSSGSASAPTLFPAAAHHHHHHGAAEQKLIS
EEDLNGAA (SEQ ID NO: 64),
GL2-FR3m having the amino acid sequence: 
LQVQLQQSGPRLVKPSGTLSLTCTVSGGSISSSSYYWGWIRQHPGKGLEWIG
YIYYSGSTYYNPSLKSRFTISRDNSKNTLYLQINSLRAEDTAVYYCARLQGAR
GYSYGYGIDYWGQGTLVTVSSGSASAPTLFPAAAHHHHHHGAAEQKLISEE
DLNGAA (SEQ ID NO: 65), and,
GL2-CDR2m having the amino acid sequence:

LQVQLQQSGPRLVKPSGTLSLTCTVSGGSISSSSYYWGWIRQHPGKGLEWIG
YIGRSGSHTNYADSVKGRVTISVDTSKNQFSLKLSSVTAADTAVYYCARLQG
ARGYSYGYGIDYWGQGTLVTVSSGSASAPTLFPAAAHHHHHHGAAEQKLIS
EEDLNGAA (SEQ ID NO: 68).

2. The V domain construct of claim 1, wherein the two Ig V domains are optionally linked by a peptide linker.

3. The V domain construct of any one of claims 1 to 2, further comprising a peptide or non-peptide tag effective to reduce or to eliminate intermolecular V domain aggregation reactions, or to facilitate delivery of the IgV domain(s) to an anatomic site of interest.

4. The V domain construct of claim3, wherein the anatomic site of interest is the blood-brain barrier.

5. The V domain construct of any one of the preceding claims, further comprising a stabilizing molecule effective to reduce the metabolic clearance of said dimeric V domain construct, wherein the stabilizing molecule is polyethylene glycol or an Fc region of an immunoglobulin.

6. A monoclonal full-length immunoglobulin-like molecule with γ, α or µ class constant domains comprising the two V domains of the Ig dimeric V domain construct of any one of claims 1 to 5 as replacements for the V domains within the heavy chain and light chain subunits.

7. A nucleotide sequence encoding the V domain construct of any one of claims 1 to 5 or the monoclonal full-length immunoglobulin-like molecule of claim6.

8. A pharmaceutical composition comprising the V domain construct of any one of claims 1-5 and/or the monoclonal full-length immunoglobulin-like molecule of claim 6.

9. The V domain construct of any one of claims 1-5 and/or the monoclonal full-length immunoglobulin-like molecule of claim 6 for use as a medicament.

## Patentansprüche

1. Isoliertes oder aufgereinigtes Konstrukt einer variablen (V-) Domäne, das die Hydrolyse von HIV gp120 katalysiert und das zwei einzelne Immunglobulin (Ig)-V-Domänen umfasst, wobei das Konstrukt Folgendes umfasst:
- die V-Domäne der leichten Kette (VL) GL2-VL mit der Aminosäuresequenz: 
ETTLTQSPATLSLSPGERATLSCRASQSVSSYLAWYQQKPGQAPRLLIYDASNR
ATGIPARFSGSGSGTDFTLTISSLEPEDFAVYYCQQRSNWPLTFGGGTKLEIKR
(SEQ ID NO: 47); und
- und eine Ig-V-Domäne der schweren Kette (VH), umfassend eine Sequenz ausgewählt aus der Gruppe bestehend aus:
GL2-FR1m mit der Aminosäuresequenz: 
LQVQLQQSGGGLVQPGRSLRLSCAASGFTFSSSSYYWGWIRQHPGKGLEWIGYI
YYSGSTYYNPSLKSRVTISVDTSKNQFSLKLSSVTAADTAVYYCARLQGARGYS
YGYGIDYWGQGTLVTVSSGSASAPTLFPAAAHHHHHHGAAEQKLISEEDLNGAA
(SEQ ID NO: 64),
GL2-FR3m mit der Aminosäuresequenz: 
LQVQLQQSGPRLVKPSGTLSLTCTVSGGSISSSSYYWGWIRQHPGKGLEWIGYI
YYSGSTYYNPSLKSRFTISRDNSKNTLYLQINSLRAEDTAVYYCARLQGARGYS
YGYGIDYWGQGTLVTVSSGSASAPTLFPAAAHHHHHHGAAEQKLISEEDLNGAA
(SEQ ID NO: 65) und
GL2-CDR2m mit der Aminosäuresequenz:

LQVQLQQSGPRLVKPSGTLSLTCTVSGGSISSSSYYWGWIRQHPGKGLEWIGYI
GRSGSHTNYADSVKGRVTISVDTSKNQFSLKLSSVTAADTAVYYCARLQGARGY
SYGYGIDYWGQGTLVTVSSGSASAPTLFPAAAHHHHHHGAAEQKLISEEDLNGA
A (SEQ ID NO: 68).

2. V-Domänen-Konstrukt nach Anspruch 1, wobei die beiden Ig-V-Domänen wahlweise durch einen Peptidlinker verknüpft sind.

3. V-Domänen-Konstrukt nach einem der Ansprüche 1 bis 2, ferner umfassend ein Peptid- oder Nicht-Peptid-Tag, das zum Verringern oder Beseitigen von intermolekularen V-Domänen-Aggregationsreaktionen oder zum Erleichtern der Abgabe der Ig-V-Domäne(n) an einen anatomischen Ort von Interesse wirksam ist.

4. V-Domänen-Konstrukt nach Anspruch 3, wobei der anatomische Ort von Interesse die Blut-Hirn-Schranke ist.

5. V-Domänen-Konstrukt nach einem der vorhergehenden Ansprüche, ferner umfassend ein stabilisierendes Molekül, das zum Verringern der metabolischen Clearance des dimeren V-Domänen-Konstrukts wirksam ist, wobei das stabilisierende Molekül Polyethylenglycol oder eine Fc-Region eines Immunglobulins ist.

6. Monoklonales immunglobulinartiges Molekül voller Länge mit konstanten Domänen der γ-, α- oder µ-Klasse, umfassend die beiden V-Domänen des dimeren Ig-V-Domänen-Konstrukts nach einem der Ansprüche 1 bis 5 als Ersetzungen für die V-Domänen in den Untereinheiten der schweren Kette und der leichten Kette.

7. Nukleotidsequenz, welche das V-Domänen-Konstrukt nach einem der Ansprüche 1 bis 5 oder das monoklonale immunglobulinartige Molekül voller Länge nach Anspruch 6 codiert.

8. Pharmazeutische Zusammensetzung, umfassend das V-Domänen-Konstrukt nach einem der Ansprüche 1 bis 5 und/oder das monoklonale immunglobulinartige Molekül voller Länge nach Anspruch 6.

9. V-Domänen-Konstrukt nach einem der Ansprüche 1 bis 5 und/oder monoklonales immunglobulinartiges Molekül voller Länge nach Anspruch 6 zur Verwendung als Medikament.

## Revendications

1. Construction à domaine variable (V) isolée ou purifiée qui catalyse l'hydrolyse de la gp120 du VIH et qui comprend deux domaines V d'immunoglobuline (Ig) individuels, dans laquelle ladite construction comprend :
- le domaine V de chaîne légère (VL) GL2-VL ayant la séquence d'acides aminés : 
ETTLTQSPATLSLSPGERATLSCRASQSVSSYLAWYQQKPGQAPRLLIYDASNR
ATGIPARFSGSGSGTDFTLTISSLEPEDFAVYYCQQRSNWPLTFGGGTKLEIKR
(SEQ ID N° : 47) ; et
- et un domaine V de chaîne lourde d'Ig (VH) comprenant une séquence sélectionnée parmi le groupe constitué de :
GL2-FRlm ayant la séquence d'acides aminés : 
LQVQLQQSGGGLVQPGRSLRLSCAASGFTFSSSSYYWGWIRQHPGKGLEWIGYI
YYSGSTYYNPSLKSRVTISVDTSKNQFSLKLSSVTAADTAVYYCARLQGARGYS
YGYGIDYWGQGTLVTVSSGSASAPTLFPAAAHHHHHHGAAEQKLISEEDLNGA
A (SEQ ID N° : 64),
GL2-FR3m ayant la séquence d'acides aminés : 
LQVQLQQSGPRLVKPSGTLSLTCTVSGGSISSSSYYWGIRQHPGKGLEWIGYIYY
SGSTYYNPSLKSRFTISRDNSKNTLYLQINSLRAEDTAVYYCARLQGARGYSYG
YGIDYWGQGTLVTVSSGSASAPTLFPAAAHHHHHHGAAEQKLISEEDLNGAA
(SEQ ID N° : 65), et
GL2-CDR2m ayant la séquence d'acides aminés :

LQVQLQQSGPRLVKPSGTLSLTCTVSGGSISSSSYYWGIRQHPGKGLEWIGYIGR
SGSHTNYADSVKGRVTISVDTSKNQFSLKLSSVTAADTAVYYCARLQGARGYS
YGYGIDYWGQGTLVTVSSGSASAPTLFPAAAHHHHHHGAAEQKLISEEDLNGA
A (SEQ ID N° : 68).

2. Construction à domaine V selon la revendication 1, dans laquelle les deux domaines V d'Ig sont liés en option par un liant peptidique.

3. Construction à domaine V selon l'une quelconque des revendications 1 à 2, comprenant en outre une étiquette peptidique ou non peptidique efficace pour réduire ou pour éliminer des réactions d'agrégation de domaine V intermoléculaire ou pour faciliter la livraison du/des domaine(s) V d'Ig à un site anatomique pertinent.

4. Construction à domaine V selon la revendication 3, dans laquelle le site anatomique pertinent est la barrière hématoencéphalique.

5. Construction à domaine V selon l'une quelconque des revendications précédentes, comprenant en outre une molécule de stabilisation efficace pour réduire la clairance métabolique de ladite construction à domaine V dimère, dans laquelle la molécule de stabilisation est le polyéthylèneglycol ou une région Fc d'une immunoglobuline.

6. Molécule monoclonale de type immunoglobuline de longueur entière avec des domaines constants de classe γ, α ou µ comprenant les deux domaines V de la construction à domaine V dimère d'Ig selon l'une quelconque des revendications 1 à 5 en tant que remplacements des domaines V au sein des sous-unités de chaîne lourde et chaîne légère.

7. Séquence nucléotidique codant pour la construction à domaine V selon l'une quelconque des revendications 1 à 5 ou la molécule monoclonale de type immunoglobuline de longueur entière selon la revendication 6.

8. Composition pharmaceutique comprenant la construction à domaine V selon l'une quelconque des revendications 1 à 5 et/ou la molécule monoclonale de type immunoglobuline de longueur entière selon la revendication 6.

9. Construction à domaine V selon l'une quelconque des revendications 1 à 5 et/ou molécule monoclonale de type immunoglobuline de longueur entière selon la revendication 6 destinée à être utilisée en tant que médicament.
